# EUROPEAN PATENT SPECIFICATION

(11) **EP 4 120 961 B1**
(45) Date of publication and mention of the grant of the patent: **29.10.2025**
(21) Application number: 21708813.7
(22) Date of filing: 02.02.2021
(51) Int. Cl.: A61F 2/24

(54) **SILK-BASED ELECTROSPUN MATERIALS FOR IMPLANT SYSTEMS AND DEVICES**
ELEKTROGESPONNENE MATERIALIEN AUF SEIDENBASIS FÜR IMPLANTATSYSTEME UND -VORRICHTUNGEN
MATÉRIAUX ÉLECTROFILÉS À BASE DE SOIE POUR SYSTÈMES ET DISPOSITIFS D'IMPLANT

(30) Priority: 28.04.2020 US 202063016835 P
(43) Date of publication of application: 25.01.2023
(73) Proprietor: Edwards Lifesciences Corporation, Irvine, CA 92614-5688 (US)
(72) Inventor: NAWALAKHE, Rupesh Gajanan, Irvine, CA 92614 (US); PAWAR, Sandip Vasant, Irvine, CA 92614 (US); CHHE, Chambory, Irvine, CA 92614 (US); LADDHA, Arpit, Santa Ana, CA 92707 (US)
(74) Representative: Venner, Julia Ann
(86) International application number: PCT/US2021/016132
(87) International publication number: WO 2021/221748

(56) References cited:
- WO-A1-2014/066724
- WO-A1-2020/123945
- US-A1- 2016 317 305

## Description

This application claims the benefit of U.S. Provisional Application No. 63/016,835, filed April 28, 2020.

### FIELD

The present disclosure concerns aspects of medical implant devices incorporating materials inclusive of a plurality of fibers that comprise an electrospun silk. The present dislcosure also concerns aspects of methods of forming such medical implant devices.

### BACKGROUND

The heart can suffer from various valvular diseases or malformations that result in significant malfunctioning of the heart and ultimately require replacement of the native heart valve with an artificial valve. Human heart valves, which include the aortic, pulmonary, mitral, and tricuspid valves, function essentially as one-way valves operating in synchronization with the pumping heart. The valves allow blood to flow downstream but block blood from flowing upstream. Diseased heart valves exhibit impairments such as narrowing the valve or regurgitation, which inhibits the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open-heart surgery. Replacement valves are being designed to be delivered through minimally invasive procedures and even percutaneous procedures. Such replacement valves often include a tissue-based valve body connected to an expandable frame that is then delivered to the native valve's annulus.

These replacement valves are often intended to at least partially block blood flow. However, a problem occurs when blood flows around the valve on the outside of the prosthesis. For example, in the context of replacement heart valves, paravalvular leakage has proven particularly challenging. An additional challenge relates to the ability of such prostheses to be secured relative to intra-luminal tissue, e.g., tissue within any body lumen or cavity, in an atraumatic manner. Further challenges arise when trying to controllably deliver and secure such prostheses in a location such as at a native mitral valve. These replacement valves are often intended to at least partially block blood flow.

Because of the drawbacks associated with conventional open-heart surgery, percutaneous and minimally-invasive surgical approaches are garnering intense attention. In one technique, a prosthetic valve is configured to be implanted in a much less invasive procedure by way of catheterization. For instance, U.S. Pat. Nos. 5,411,522 and 6,730,118, describe collapsible transcatheter heart valves that can be percutaneously introduced in a compressed state on a catheter and expanded in the desired position by balloon inflation or by utilization of a self-expanding frame or stent. In yet another example, U.S. U.S. Publication Nos. 2014/0277390, 2014/0277422, 2014/0277427, and 2015/0328000, and 2019/0328515, describe heart valve prostheses for replacing a native mitral valve, including a self-expanding frame with a plurality of anchoring members that are designed be deployed within a body cavity and prevent axial flow of fluid around an exterior of the prosthesis.

However, the manufacturing of such implantable devices can be cumbersome and expansive and often limiting. For example, the textile used as a part of the paravalvular leakage sealing material is often formed from woven or knitted fabric comprising medical-grade polyester (PET) filaments. The process requiring to produce such a fabric is time-consuming, and the resulting product has multiple limitations. For example, the PET filaments used for such construction are limited to larger diameter filaments. Currently, the minimum applicable diameter of PET filament is about 10 microns that can affect other properties of the formed textile, such as a textile surface area, smoothness of the surface, tensile properties of the fabric, and the like. In addition, the PET-based fabrics are not biodegradable and/or bioresorbable that further limits the use of such fabrics.

Also, the manufacturing of other parts of the valve has additional challenges and limitations. For example, valve leaflets are often formed from the material that is obtained from a living tissue that makes such production more complicated and also expansive.

Thus, there is still a need for implantable devices that comprise materials having the desired mechanical and chemical properties that are easy to manufacture. These needs and others are at least partially satisfied by the present disclosure.

US 2016/317305 A1 relates to an implantable prosthetic valve that is radially collapsible to a collapsed configuration and radially expandable to an expanded configuration comprises an annular frame having an inflow end and an outflow end, a leaflet structure positioned within the frame and secured thereto, and a laminate sealing member comprising an encapsulating material. The laminate sealing member has a main portion that encapsulates at least a portion of the frame and an end portion extending from the inflow end of the frame, and the end portion of the laminate sealing member is folded to form a cuff adjacent the inflow end of the frame and secured to the main portion of the laminate sealing member.

### SUMMARY

Some of the aspects of the present disclosure relate to implantable prosthetic valves. The invention as defined in claim 1 relates to an implantable prosthetic valve comprising: an annular frame having an inner surface and an outer surface wherein the frame has an inflow end and an outflow end and a central longitudinal axis extending from the inflow end to the outflow end; a leaflet structure positioned within the frame; an inner skirt positioned along the inner surface of the frame; at least one outer skirt positioned around the outer surface of the frame; wherein at least a portion of one of the leaflet structure, the inner skirt, or the at least one outer skirt comprises a material comprising a plurality of fibers, wherein at least one fiber of the plurality of fibers comprises
electrospun silk; and wherein the implantable prosthetic valve is radially collapsible to a collapsed configuration and radially expandable to an expanded configuration.

In yet other aspects, disclosed is the implantable prosthetic valve, wherein at least a portion of the inner skirt comprises the material comprising the plurality of fibers comprising the electrospun silk, and wherein the material present in the at least a portion of the inner skirt is a first material. In one aspect, disclosed is the implantable prosthetic valve of the preceding aspects, wherein at least a portion of the outer skirt comprises the material comprising the plurality of fibers comprising the electrospun silk, and wherein the material present in the at least a portion of the outer skirt is a second material. In yet another aspect, disclosed is the implantable prosthetic valve **of the preceding aspects,** wherein at least a portion of the leaflet structure comprises the material comprising the plurality of fibers comprising the electrospun silk, and wherein the material present in the at least a portion of the leaflet structure is a third material. Also disclosed herein are aspects wherein the first, second, and third materials are the same or different.

**Still disclosed herein are the aspects where** each fiber of the plurality of fibers has a first extending direction and a plurality of undulations. **In some aspects,** the first extending direction can comprise a circumferential direction, a radial direction, or a combination thereof. **In still further aspects, the described** plurality of undulations can be present in the collapsed configuration. **In still further aspects,** the plurality of undulations can be configured to straighten when the implantable prosthetic valve is in the expanded configuration.

**In one aspect, disclosed is the** implantable prosthetic valve where the valve further comprises an adhesive material disposed between at least a portion of the annular frame and at least a portion of the outer skirt and/or between at least a portion of the annular frame and at least a portion of the inner skirt. While in other aspects, **disclosed is the** implantable prosthetic valve where at least a portion of the inner skirt is attached to the annular frame by direct electrospinning of the plurality of fibers on at least a portion of the inner surface of the annular frame. Still in further aspects, **disclosed is the** implantable prosthetic valve where at least a portion of the outer skirt is attached to at least a portion of the annular frame by direct electrospinning of the plurality of fibers on at least a portion of the outer surface of the annular frame.

Still, further, the implantable prosthetic valves of any one of the preceding aspects where at least a portion of the plurality of fibers have a random orientation are also disclosed. While in other aspects, at least a portion of the plurality of fibers can have a predetermined aligned orientation.

In some exemplary aspects, the plurality of fibers can further comprise other than silk materials. In some exemplary aspects, the plurality of fibers can further comprise a resorbable material, a non-resorbable material, or any combination thereof. For example, in some aspects, the plurality of fibers can further comprise thermoplastic polyurethane (TPU), polyurethane (PU); implantable elastane polymer, polyurethane (PU); implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

Still further disclosed is an aspect where at least a portion of the plurality of fibers comprises a bicomponent fiber. It is understood that in such exemplary aspects, the bicomponent fiber can comprise a side-by-side configuration, a sheath-core configuration, an islands-in-the-sea configuration, a tri-lobal, a segmented pie configuration, or any combination thereof. While in some exemplary and unlimiting aspects, the bicomponent fiber can comprise the sheath-core configuration.

In some aspects, a sheath and/or a core of the bicomponent fiber comprises a resorbable material, non-resorbable material, or any combination thereof In certain aspects, a sheath of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and wherein a core of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof. While is still further exemplary aspects, a sheath of the bicomponent fiber can comprise silk, while a core of the bicomponent fiber can comprise one or more of thermoplastic polyurethane (TPU), polyurethane (PU) implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

Also disclosed herein are aspects where the plurality of fibers can have an average diameter from about 3 nm to about 15,000 nm. While in other aspects, at least a portion of the first material, and/or the second material, and/or third material can exhibit porosity. In such exemplary and unlimiting aspects, the at least a portion of the first material, and/or the second material, and/or third material can have an average pore size from about 100 nm to about 100 µm. In still further aspects, wherein the first material and/or the second material, and/or third material can comprise a plurality of layers, wherein each of the plurality of layers comprises electrospun silk, and wherein each of the plurality of layers is disposed on each other. In such exemplary aspects, at least a first portion of the plurality of layers has a surface area that is substantially smaller than a surface area of a second portion of the plurality of layers surface area,

In still further aspects, the first material and/or the second material, and/or third material used in any of the disclosed above implantable prosthetic devices can exhibit a tensile strength from greater than 0 MPa to about 20 MPa. While in other aspects, the first material and/or the second material, and/or third material can exhibit an elongation at break from greater than 0% to about 600%. In yet still further aspects, the first material and/or the second material, and/or third material can exhibit a water contact angle from about 0° to about 180°.

Also disclosed are the aspects where at least a portion of the annular frame is surface modified. In certain and unlimiting aspects, at least a portion of the annular frame is plasma treated. While in other aspects, at least a portion of the inner skirt can be surface modified. In such exemplary aspects, at least a portion of the inner skirt comprising the first material is plasma treated. While in still further aspects, at least a portion of the outer skirt can be surface modified. In such exemplary aspects, at least a portion of the outer skirt comprising the second material can be plasma treated. Still further, in some aspects, also at least a portion of the leaflet system can be surface modified. In such exemplary aspects, wherein at least a portion of the leaflet structure comprising the third material is plasma treated.

Further disclosed herein is an aspect where the first material and/or the second material, and/or third material is at least partially biodegradable. While in other aspects, the first material and/or the second material, and/or third material is at least partially bioresorbable. In still further aspects, the first material and/or the second material, and/or third material can be both at least partially biodegradable and at least partially bioresorbable. In yet further aspects, the first material and/or the second material, and/or third material as described in any preceding aspects, can be at least partially degradable. In still further aspects, the first material and/or the second material, and/or third material as described in any preceding aspects, can be a scaffold material.

In still further aspects, the disclosed herein implanted valves can have at least a portion of the inner skirt further comprising a first perforated material having a first surface facing the annular frame and an opposite second surface and wherein the first material can comprise the plurality of fibers comprising the electrospun silk is disposed on the first surface and/or the second surface of the first perforated material.

In yet other aspects, at least a portion of the outer skirt can further comprise a second perforated material having a first surface facing the annular frame and an opposite second surface and wherein the second material comprises the plurality of fibers comprising the electrospun silk is disposed on the first surface and/or the second surface of the second perforated material.

Also disclosed are aspects, where at least a portion of the leaflet structure comprises a third perforated material having a first surface facing the annular frame and an opposite second surface and wherein the third material comprising the plurality of fibers comprising the electrospun silk is disposed on the first surface and/or the second surface of the third perforated material.

In addition or in the alternative, also disclosed are aspects where at least a portion of the leaflet structure comprises a third perforated material having a first surface facing the annular frame and an opposite second surface and wherein the third material comprising the plurality of fibers comprising the electrospun silk is disposed on the first surface and/or the second surface of the third perforated material.

Still further, in some aspects, the first perforated material, the second perforated material and/or the third perforated material can be the same or different.

Also disclosed are aspects where at least a portion of the first surface of the first material comprises a first auxiliary layer. While in the alternative or additional aspects, at least a portion of the second surface of the first material comprises a first auxiliary layer.

In some aspects disclosed is the implantable prosthetic valve where the first auxiliary layer present on the second surface of the first material is the same or different as the first auxiliary layer present on the first surface of the first material.

Yet in other aspects, wherein at least a portion of the first surface of the second material comprises a second auxiliary layer.

Still further disclosed are aspects where at least a portion of the second surface of the second material comprises a second auxiliary layer.

Also disclosed are aspects where the second auxiliary layer present on the second surface of the second material is the same or different as the second auxiliary layer present on the first surface of the second material.

Still further disclosed are aspects where at least a portion of the first surface of the third material comprises a third auxiliary layer. While in other aspects, at least a portion of the second surface of the third material comprises a third auxiliary layer.

In still further aspects, the third auxiliary layer present on the second surface of the third material is the same or different as the third auxiliary layer present on the first surface of the third material.

While in still further aspects, each of the first, second or third auxiliary layers can be the same or different.

In some aspects, the first, second, and/or third perforated material can comprise a porous fabric or membrane, wherein the porous fabric or membrane comprises one or more biocompatible polymers that are resorbable, non-resorbable or a combination thereof. In some exemplary and unlimiting aspects, the first, second, and/or third perforated material can comprise a porous fabric or membrane, wherein the porous fabric or membrane comprises one or more biocompatible polymers selected from polyethylene, polypropylene, polymethylmethacrylate (PMMA), polystyrene (PS), polytetrafluoroethylene (PTFE), thermoplastic polyurethane (TPU), polyurethane (PU), implantable elastane polymer, polyamide, polyethylene terephthalate (PET), polyethersulfone, poly-lactic-co-glycolic acid (PLGA) or a combination thereof, or natural/regenerated fibers selected from cotton, silk, linen, cellulose acetate, collagen, or a combination thereof.

In yet other aspects, the first, second, and/or third auxiliary layer configured to impart to at least a portion of the first, second, and/or third material hydrophobic or hydrophilic properties, elastomeric properties, mechanical resilience, adhesive properties, tissue-in-growth inhibition, or any combination thereof.

In still further aspects, the first, second, and/or third auxiliary layer comprises one or more of resorbable, non-resorbable, or a combination thereof materials. In addition or in the alternative also disclosed are aspects where the first, second, and/or third auxiliary layer comprises one or more thermoplastic polyurethane (TPU), polyurethane (PU), implantable elastane polymer, or polyethylene, polypropylene, polymethylmethacrylate (PMMA), polystyrene (PS), polytetrafluoroethylene (PTFE), polyamide, polyethylene terephthalate (PET), polyethersulfone, poly-lactic-co-glycolic acid (PLGA).

In some further aspects, at least a portion of the inner skirt further comprises at least two layers of the first perforated material, wherein the first material comprising the plurality of fibers comprising the electrospun silk is disposed between the two layers of the first perforated material, and wherein at the two layers of the first perforated material are at least partially coupled to each other.

While in other aspects, at least a portion of the outer skirt can further comprise at least two layers of the second perforated material, wherein the second material comprising the plurality of fibers comprising the electrospun silk is disposed between the two layers of the second perforated material and wherein at the two layers of the second perforated material are at least partially coupled to each other.

Also disclosed are aspects where at least a portion of the leaflet structure further comprises at least two layers of the third perforated material, wherein the third material comprising the plurality of fibers comprising the electrospun silk is disposed between the two layers of the third perforated material, and wherein at the two layers of the third perforated material are at least partially coupled to each other.

In still further aspects, wherein at least a portion of the second surface of the first material is disposed on the first surface of the first perforated material While in other aspects, at least a portion of the first surface of the first material is disposed on the second surface of the first perforated material. In yet still further aspects, at least a portion of the second surface of the second material is disposed on the first surface of the second perforated material.

Still, in further exemplary and unlimiting aspects, at least a portion of the first surface of the second material is disposed on the second surface of the second perforated material. Yet, in other aspects, at least a portion of the second surface of the third material is disposed on the first surface of the third perforated material

In some aspects, at least a portion of the first surface of the third material is disposed on the second surface of the third perforated material.

While in other aspects, wherein at least a portion of the first auxiliary layer and the first perforated material is coupled to each other.

Still further disclosed are aspects where at least a portion of the second auxiliary layer and the second perforated material are coupled to each other. While in other aspects, at least a portion of the third auxiliary layer and the third perforated material are coupled to each other.

Also disclosed herein, in some aspects, is an article comprising a material comprising a plurality of fibers, wherein at least one fiber of the plurality of fibers comprises electrospun silk, wherein the article has a collapsed configuration and an expanded configuration, and wherein the article is a part of an implantable device. In such exemplary aspects, the article is a paravalvular leak sealing article. In still further aspects, the paravalvular leak sealing article can comprise an inner skirt comprising a first material comprising a plurality of fibers comprising an electrospun silk and where the inner skirt is configured to be positioned on at least a portion of an inner surface of an annular frame of the implantable prosthetic device, wherein the first material has a first surface facing the annular frame and an opposite second surface. While in still further aspects, the paravalvular leak sealing article can comprise an outer skirt comprising a second material comprising a plurality of fibers comprising an electrospun silk and where the outer skirt is configured to be positioned on at least a portion of an outer surface of an annular frame of the implantable prosthetic device, wherein the second material has a first surface facing the annular frame and an opposite second surface.

Still further also disclosed herein are aspects where the article can comprise a leaflet structure comprising a third material comprising a plurality of fibers comprising an electrospun silk and where the leaflet structure is configured to be positioned within at least a portion of an annular frame of the implantable prosthetic device, wherein the third material has a first surface facing the annular frame and an opposite second surface. It is understood that in the aspects disclosed herein, the disclosed material comprises the first material, or the second material, or the third material, or a combination thereof. In yet further aspects, the disclosed herein the first, the second, and the third materials can be the same or different.

In still further aspects, each fiber of the plurality of fibers has a first extending direction and a plurality of undulations. Further, in such exemplary aspects, the first extending direction can comprise a circumferential direction, a radial direction, or a combination thereof. Still, further, the plurality of undulations, as described in any preceding aspect, are present in the collapsed configuration. It is further understood that in such aspects, the plurality of undulations are configured to straighten when the article is in the expanded configuration.

In certain aspects, at least a portion of the inner skirt is attached to at least a portion of the annular frame by direct electrospinning of the plurality of fibers, While in other exemplary aspects, at least a portion of the outer skirt is attached to at least a portion of the annular frame by direct electrospinning of the plurality of fibers.

Also disclosed are articles where wherein the plurality of fibers further comprise a resorbable material, a non-resorbable material, or a combination thereof. In some aspects, at least a portion of the plurality of fibers has a random orientation. While in other aspects, at least a portion of the plurality of fibers has a predetermined aligned orientation. In such aspects, the articles disclosed in any of the preceding aspects can also comprise a plurality of fibers, where the plurality of fibers further comprises thermoplastic polyurethane (TPU), polyurethane (PU) implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

In still further exemplary aspects, at least a portion of the plurality of fibers can comprise a bicomponent fiber. It is understood that the bicomponent fiber can comprise any known configuration. In some exemplary and unlimiting aspects, the bicomponent fiber can comprise a side-by-side configuration, a sheath-core configuration, a tri-lobal, an islands-in-the-sea configuration, a segmented pie configuration, or any combination thereof. While in one aspect, the bicomponent fiber comprises the sheath-core configuration.

In certain aspects, a sheath and/or core of the bicomponent fiber comprises a resorbable material, a non-resorbable material, or a combination thereof. In yet other aspects, a sheath of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and wherein a core of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

While is still further exemplary aspects, a sheath of the bicomponent fiber can comprise silk, while a core of the bicomponent fiber can comprise one or more of thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

In still further aspects, the article, as described in any preceding aspects, can comprise a plurality of fibers, wherein the plurality of fibers have an average diameter from about 3 nm to about 15,000 nm. While in other exemplary aspects, at least a portion of the first material and/or the second material and/or the third material can exhibit porosity. While in still further exemplary aspects, the at least a portion of the first material and/or the second material and/or the third material can have an average pore size from about 100 nm to about 100 µm.

Also disclosed herein are aspects of the article, where at least a portion of the first material and/or the second material and/or the third material comprise a plurality of layers, wherein each of the plurality of layers comprises electrospun silk, and wherein each of the plurality of layers is disposed on each other. In such exemplary aspects, at least a first portion of the plurality of layers has a surface area that is substantially smaller than a surface area of a second portion of the plurality of layers surface area.

Still further disclosed herein are aspects of the article, where at least a portion of the first material and/or the second material and/or the third material can exhibit tensile strength from greater than 0 MPa to about 20 MPa. While further disclosed herein are aspects of the article, where at least a portion of the first material and/or the second material and/or the third material can exhibit elongation at break from greater than 0% to about 600%. In still further aspects, at least a portion of the first material and/or the second material and/or the third material can exhibit a water contact angle from about 0° to about 180°. It is understood that also disclosed herein are aspects of the article, where at least a portion of the first material and/or the second material and/or the third material can exhibit the disclosed above tensile strength, elongation, and/or a water contact angle.

In still further aspects, disclosed herein are the articles as described in any one of preceding aspects, where at least a portion of the first material, and/or the second material, and/or the third material is surface modified by any known in the art methods. In some exemplary aspects, at least a portion of the first material comprising the plurality of fibers is plasma treated. While in other exemplary aspects, at least a portion of the second material is plasma treated. While in still further exemplary aspects, at least a portion of the third material is plasma treated.

In certain aspects, at least a portion of the first material and/or the second material and/or the third material can be at least partially biodegradable. While in other aspects, at least a portion of the first material and/or the second material and/or the third material can be at least partially bioresorbable. While in still further aspects, at least a portion of the first material and/or the second material and/or the third material can be at least partially degradable. It is understood, however, that also disclosed herein are the aspects where at least a portion of the first material and/or the second material and/or the third material is at least partially biodegradable and/or at least partially bioresorbable and/or at least partially degradable. In yet further aspects, at least a portion of the first material and/or the second material and/or the third material as described in any preceding aspects can be also be configured to be a scaffold material.

**Also disclosed** herein articles, where at least a portion of the inner skirt further comprises a first perforated material having a first surface facing the annular frame and an opposite second surface and wherein the first material comprising the plurality of fibers comprising the electrospun silk is disposed on the first surface and/or the second surface of the first perforated material,

In still further aspects, disclosed are articles where at least a portion of the outer skirt further comprises a second perforated material having a first surface facing the annular frame and an opposite second surface and wherein the second material comprising the plurality of fibers comprising the electrospun silk is disposed on the first surface and/or the second surface of the second perforated material.

Also disclosed are articles, where at least a portion of the leaflet structure comprises a third perforated material having a first surface facing the annular frame and an opposite second surface and wherein the third material comprising the plurality of fibers comprising the electrospun silk is disposed on the first surface and/or the second surface of the third perforated material.

In some additional or alternative aspects, the first perforated material, the second perforated material and/or the third perforated material can be the same or different.

While yet in other aspects, disclosed are articles where at least a portion of the first surface of the first material comprises a first auxiliary layer. While in still other aspects, at least a portion of the second surface of the first material comprises a first auxiliary layer. **Also disclosed are articles where** the first auxiliary layer present on the second surface of the first material is the same or different as the first auxiliary layer present on the first surface of the first material.

In addition or in the alternatives disclosed are articles where at least a portion of the first surface of the second material comprises a second auxiliary layer. In some exemplary aspects, at least a portion of the second surface of the second material comprises a second auxiliary layer. In still further aspects, the second auxiliary layer present on the second surface of the second material is the same or different as the second auxiliary layer present on the first surface of the second material.

In addition or in the alternatives disclosed are articles where at least a portion of the first surface of the third material comprises a third auxiliary layer. In some exemplary aspects, at least a portion of the second surface of the third material comprises a third auxiliary layer. In still further aspects, the third auxiliary layer present on the second surface of the third material is the same or different as the third auxiliary layer present on the first surface of the third material.

Still, further, each of the first, second or third auxiliary layers can be the same or different

Also disclosed herein are articles where the first, second, and/or third perforated material comprises a porous fabric or membrane, wherein the porous fabric or membrane comprises a resorbable material, a non-resorbable material, or a combination thereof. In yet other aspects, the first, second, and/or third perforated material comprises a porous fabric or membrane, wherein the porous fabric or membrane comprises one or more biocompatible polymers selected from polyethylene, polypropylene, polymethylmethacrylate (PMMA), polystyrene (PS), polytetrafluoroethylene (PTFE), thermoplastic polyurethane (TPU), polyurethane (PU), implantable elastane polymer, polyamide, polyethylene terephthalate (PET), polyethersulfone, poly-lactic-co-glycolic acid (PLGA) or a combination thereof or natural/regenerated fibers selected from cotton, silk, linen, cellulose acetate, collagen, or a combination thereof.

In addition or in the alternative, disclosed are articles where the first, second, and/or third auxiliary layer configured to impart to at least a portion of the first, second, and/or third material hydrophobic or hydrophilic properties, elastomeric properties, mechanical resilience, adhesive properties, tissue-in-growth inhibition, or any combination thereof.

In yet further aspects, disclosed are articles where the first, second, and/or third auxiliary layer comprises one or more of a resorbable material, a non-resorbable material, or a combination thereof. Yet in other aspects, the first, second, and/or third auxiliary layer comprises one or more thermoplastic polyurethane (TPU), polyurethane (PU), implantable elastane polymer, or polyethylene, polypropylene, polymethylmethacrylate (PMMA), polystyrene (PS), polytetrafluoroethylene (PTFE), polyamide, polyethylene terephthalate (PET), polyethersulfone, poly-lactic-co-glycolic acid (PLGA).

Also disclosed are articles where at least a portion of the inner skirt further comprises at least two layers of the first perforated material, wherein the first material comprising the plurality of fibers comprising the electrospun silk is disposed between the two layers of the first perforated material, and wherein at the two layers of the first perforated material are at least partially coupled to each other.

In some aspects, disclosed are articles where at least a portion of the outer skirt further comprises at least two layers of the second perforated material, wherein the second material comprising the plurality of fibers comprising the electrospun silk is disposed between the two layers of the second perforated material and wherein at the two layers of the second perforated material are at least partially coupled to each other.

While in other aspects, disclosed are articles where at least a portion of the leaflet structure further comprises at least two layers of the third perforated material, wherein the third material comprising the plurality of fibers comprising the electrospun silk is disposed between the two layers of the third perforated material; and wherein at the two layers of the third perforated material are at least partially coupled to each other.

In some exemplary and unlimiting aspects disclosed are articles where at least a portion of the second surface of the first material is disposed on the first surface of the first perforated material. Yet, in other aspects, at least a portion of the first surface of the first material is disposed on the second surface of the first perforated material.

In still further aspects, at least a portion of the second surface of the second material is disposed on the first surface of the second perforated material. While in other aspects, at least a portion of the first surface of the second material is disposed on the second surface of the second perforated material. While in still further aspects, at least a portion of the second surface of the third material is disposed on the first surface of the third perforated material.

Also disclosed are aspects directed to the articles having at least a portion of the first surface of the third material be disposed on the second surface of the third perforated material.

In some aspects, at least a portion of the first auxiliary layer and the first perforated material are coupled to each other. While in other aspects, at least a portion of the second auxiliary layer and the second perforated material are coupled to each other. While in still further aspects, at least a portion of the third auxiliary layer and the third perforated material is coupled to each other.

Also disclosed herein are methods of forming an implantable prosthetic valve. According to the invention as defined in claim 8, the method comprises: a) providing an annular frame having an inner surface and an outer surface wherein the frame has an inflow end and an outflow end and a central longitudinal axis extending from the inflow end to the outflow end; b) forming an inner skirt comprising a first material having a first surface and an opposite second surface and comprising a plurality of fibers, wherein at least one fiber of the plurality of fibers comprises electrospun silk; c) forming an outer skirt comprising a second material having a first surface and an opposite second surface and comprising a plurality of fibers, wherein at least one fiber of the plurality of fibers comprises electrospun silk; d) attaching the inner skirt to at least a portion of the inner surface of the annular frame and attaching the outer skirt to at least a portion of the outer surface of the annular frame, wherein the implantable prosthetic valve is radially collapsible to a collapsed configuration and radially expandable to an expanded configuration.

In some aspects, the step of forming the inner skirt and the step of attaching the inner skirt can occur simultaneously. Yet, in other aspects, the step of forming the inner skirt can occur prior to the step of attaching.

In addition or in the alternative disclosed are methods where the step of forming the outer skirt and the step of attaching the outer skirt occurs simultaneously. While in other aspects, the step of forming the outer skirt can occur prior to the step of attaching.

In some methods, the step of attaching the inner skirt occurs before or after the step of attaching the outer skirt.

In addition or in the alternative, the methods disclosed herein further comprise positioning a leaflet structure comprising a third material having a first surface and an opposite second surface and comprising a plurality of fibers, wherein at least one fiber of the plurality of fibers comprises an electrospun silk within at least a portion of the annular frame.

In some aspects, the step of positioning the leaflet structure can occur before or after the step of forming the inner skirt and/or outer skirt.

Also disclosed are the methods where the first material, the second, and the third material are the same or different.

In addition or in the alternative disclosed are methods where the step of simultaneously forming and attaching the inner skirt to the at least a portion of the inner surface of the annular frame comprises forming the first material by directly electrospinning at least a portion of the plurality of fibers through at least one spinneret from a first solution comprising a first predetermined concentration of a silk fibroin at a predetermined extrusion rate at the at least a portion of the inner surface of the annular frame.

In further aspects disclosed are the methods where the step of forming the first material comprises electrospinning at least a portion of the plurality of fibers through at least one spinneret from a first solution comprising a first predetermined concentration of a silk fibroin at a predetermined extrusion rate on a first predetermined mandrel.

Also disclosed are methods where the step of attaching comprises i) shaping the first material to a predetermined dimension and ii) attaching the first material to the at least a portion of the inner surface of the annular frame.

In addition or in the alternative disclosed are methods where the step of simultaneously forming and attaching the outer skirt to the at least a portion of the outer surface of the annular frame comprises forming the second material by directly electrospinning at least a portion of the plurality of fibers through at least one spinneret from a second solution comprising a second predetermined concentration of a silk fibroin at a predetermined extrusion rate the at least a portion of the outer surface of the annular frame.

Yet, in other aspects, the step of forming the second material comprises electrospinning at least a portion of the plurality of fibers through at least one spinneret from a second solution comprising a second predetermined concentration of a silk fibroin at a predetermined extrusion rate on a second predetermined mandrel.

In addition or in the alternative disclosed are methods where the step of attaching comprises i) shaping the second material to a predetermined dimension and ii) attaching the second material to the at least a portion of the outer surface of the annular frame.

In still further methods, the third material can be formed by the electrospinning of the plurality of fibers on a third predetermined mandrel from a third solution comprising a third predetermined concentration of a silk fibroin at a predetermined extrusion rate. In some methods, the third material can be laser cut to form the leaflet structure

In some exemplary and unlimiting methods, prior to forming the inner skirt and/or the outer skirt, at least a portion of the annular frame is plasma treated. While in other methods, prior to the step attaching the inner skirt and/or the outer skirt to the at least a portion of the inner surface and/the outer surface of the annular frame, respectively, an adhesive material is applied to the at least a portion of the inner surface and/or the outer surface of the annular frame.

In some aspects in addition or in the alternative to any one of the preceding aspects, during the electrospinning of the at least a portion of the plurality of fibers to form the first material, the at least a portion of the inner surface of the annular frame can be positioned at a first predetermined distance from the at least one extrusion spinneret. Yet in other aspects, during the electrospinning of the at least a portion of the plurality of fibers to form the second material, the at least a portion of the outer surface of the annular frame can be positioned at a second predetermined distance from the at least one extrusion spinneret.

In some exemplary aspects, the at least one extrusion spinneret is positioned outside of the annular frame.

While in other aspects, the at least one extrusion spinneret is positioned within at least a portion of an inner space of the annular frame, wherein the inner space is defined by a circumference of the inner surface of the annular frame. In such exemplary and unlimiting aspects, the methods can further comprise at least one additional extrusion spinneret that is positioned outside of the annular frame.

In such exemplary methods, the electrospinning can occur simultaneously from the at least one extrusion spinneret positioned within the at least a portion of the inner space of the annular frame and from the at least one additional spinneret that is positioned outside of the annular frame. While in other methods, the electrospinning can occur first from the at least one extrusion spinneret positioned within the at least a portion of the inner space of the annular frame and then from the at least one additional spinneret that is positioned outside of the annular frame.

In yet other methods, the electrospinning can occur first from the at least one additional spinneret that is positioned outside of the annular frame and then from the at least one extrusion spinneret positioned within the at least a portion of the inner space of the annular frame. In still further aspects, the electrospinning is performed by cycling.

In some aspects, the at least one extrusion spinneret positioned within the at least a portion of the inner space of the annular frame and the at least one additional spinneret positioned outside of the annular frame has an extrusion rate that is the same or different. While in other aspects, each of the at least one extrusion spinneret positioned within the at least a portion of the inner space of the annular space and the at least one additional spinneret positioned outside are configured to electrospun a plurality of fibers from a solution having the same or different concentration of a silk fibroin.

Also disclosed are the methods where the at least one extrusion spinneret positioned within the at least a portion of the inner space of the annular frame at a third distance from the annular frame and is configured to be moved within the inner space of the annular frame. In still further aspects, the third predetermined distance from the annular frame is adjustable.

In some aspects, the at least one additional spinneret positioned outside of the annular frame is positioned at a fourth predetermined distance from the annular frame. In yet further aspects, the fourth predetermined distance from the annular frame is adjustable. In still further aspects, the first, second, third and/or fourth predetermined distances are the same or different.

In some aspects, disclosed are methods where the plurality of fibers formed by the at least one extrusion spinneret positioned within the at least a portion of the inner space of the annular frame and the at least one additional spinneret positioned outside of the annular frame are consolidated.

In some additional and unlimiting aspects, during the electrospinning of the at least a portion of the plurality of fibers to form the third material, the at least a portion of the third predetermined mandrel can be positioned at a third predetermined distance from the at least one extrusion spinneret.

Also disclosed are methods, where during the electrospinning of the at least a portion of the plurality of fibers to form the first material and/or the second material and/or the third material, the at least a portion of the inner surface of the annular frame and/or the at least a portion of the outer surface of the annular frame and/or the at least a portion of the first, second and/or third predetermined mandrels are positioned at a distance from the at least one extrusion spinneret such that the distance is varied during the electrospinning to form one or more plurality of layers within at least a portion of the first material and/or the second material and/or the third material.

In some methods, at least a portion of the annular frame is positioned on a rotational drum configured to rotate at a predetermined speed. While in other methods, the first, second, and/or third predetermined mandrels are configurated to be rotational or stationary.

Also disclosed are methods where a first predetermined voltage is applied between the rotational drum and the at least one spinneret. Yet, in other aspects, a second predetermined voltage is applied between the first, second and/or third predetermined mandrels and the at least one spinneret.

In some methods, the at least one spinneret comprises a needle.

Yet, in other methods, the at least one spinneret is a part of an assembly comprising a plurality of spinnerets. In such exemplary and unlimiting methods, the assembly can comprise a plurality of needle-less spinnerets.

Also disclosed are methods where the plurality of fibers present in the first material and/or the second material and/or the third material comprise a first extending direction and a plurality of undulations. In such exemplary methods, the first extending direction can comprise a circumferential direction, a radial direction, or a combination thereof. In still further aspects, the plurality of undulations are present in the collapsed configuration of the implantable prosthetic valve. While in other aspects, the plurality of undulations are configured to straighten when the implantable prosthetic valve is in the expanded configuration.

In some methods, at least a portion of the plurality of fibers present in the first material and/or the second material and/or the third material has a random orientation. While in other methods, at least a portion of the plurality of fibers present in the first material and/or the second material and/or the third material has a predetermined aligned orientation.

In addition or in the alternative disclosed are methods, where the plurality of fibers present in the first material and/or the second material and/or the third material further comprise a resorbable material, a non-resorbable material, or a combination thereof. In yet other methods, the plurality of fibers present in the first material and/or the second material and/or the third material further comprise thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

In some methods, wherein the plurality of fibers are disposed by electrospinning through the at least one spinneret from the first solution and/or the second solution and/or the third solution further comprising a predetermined concentration of a resorbable material, a non-resorbable material, or a combination thereof. In such exemplary and unlimiting aspects, the plurality of fibers are disposed by electrospinning through the at least one spinneret from the first solution and/or the second solution and/or the third solution, further comprising a predetermined concentration of the thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof at a predetermined extrusion rate.

While in other methods, at least a portion of the plurality of fibers present in the first material and/or the second material, and/or the third material comprises a bicomponent fiber. In such exemplary and unlimiting methods, the bicomponent fiber comprises a side-by-side configuration, a sheath-core configuration, a tri-lobal, an islands-in-the-sea configuration, a segmented pie configuration, or any combination thereof.

In some aspects, the bicomponent fiber can comprise the sheath-core configuration. For example, disclosed are methods where wherein a sheath and a core of the bicomponent fiber comprises a resorbable material, a non-resorbable material, or a combination thereof. Yet in other methods, a sheath of the bicomponent fiber comprises one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and wherein a core of the bicomponent fiber comprises one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

While also disclosed are methods where a sheath of the bicomponent fiber comprises silk and wherein a core of the bicomponent fiber comprises one or more of thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

In some aspects, the bicomponent fibers are disposed by electrospinning through at least two concentric spinnerets, wherein an outer spinneret is configured to extrude a sheath fiber from a fourth solution comprising a fourth predetermined concentration of silk fibroin, and wherein an inner spinneret is configured to extrude a core fiber from a fifth solution comprising a predetermined concentration of resorbable material, a non-resorbable material, or a combination thereof. In such exemplary and unlimiting aspects, the bicomponent fibers can be disposed by electrospinning through at least two concentric spinnerets, wherein an outer spinneret is configured to extrude a sheath fiber from a fourth solution comprising a fourth predetermined concentration of silk fibroin, and wherein an inner spinneret is configured to extrude a core fiber from a fifth solution comprising a predetermined concentration of thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof at a predetermined extrusion rate.

Also disclosed are methods where the plurality of fibers present in the first material and/or the second material and/or the third material have an average diameter from about 3 nm to about 15,000 nm.

In some methods, at least a portion of the first material and/or the second material and/or the third material exhibit porosity. In such exemplary methods, the at least a portion of the first material and/or the second material, and/or third material have an average pore size from about 100 nm to about 100 µm.

In addition or in the alternative, disclosed are methods where the first material and/or the second material, and/or third material comprise a plurality of layers, wherein each of the plurality of layers comprises electrospun silk, and wherein each of the plurality of layers is disposed on each other.

In some aspects disclosed are methods where the at least a first portion of the plurality of layers has a surface area that is substantially smaller than a surface area of a second portion of the plurality of layers surface area.

Yet in other aspects disclosed are methods, where the first material and/or the second material and/or the third material exhibit tensile strength from greater than 0 MPa to about 20 MPa. In still further aspects, the first material and/or the second material and/or the third material can exhibit elongation at break from greater than 0% to about 600%. While still in further aspects, the first material and/or the second material and/or the third material exhibit a water contact angle from about 0° to about 180°.

In some aspects, at least a portion of the first material and/or the second material and/or the third material is biodegradable. Yet, in other aspects, at least a portion of the first material and/or the second material and/or the third material is bioresorbable. Still, in other aspects, at least a portion of the first material and/or the second material and/or the third material is degradable. While still in further aspects, at least a portion of the first material and/or the second material and/or the third material is configured to be a scaffold material.

In some additional or alternative aspects, at least a portion of the plurality of fibers at present in the first material and/or the second material and/or the third material is plasma treated after electrospinning.

Also disclosed are aspects where at least a portion of the formed first material is disposed on a first perforated material having a first surface and an opposite second surface, prior to the step of attaching, and wherein the first material is disposed on the first surface and/or the second surface of the first perforated material.

In some methods disclosed herein, the step of attaching comprises coupling the first surface of the first perforated material to at least a portion of the annular frame.

While in other methods, at least a portion of the formed second material is disposed on a second perforated material having a first surface and an opposite second surface, prior to the step of attaching, and wherein the second material is disposed on the first surface and/or the second surface of the second perforated material. In such exemplary and unlimiting methods, the step of attaching comprises coupling the first surface of the second perforated material to at least a portion of the annular frame.

Also disclosed are the methods where at least a portion of the leaflet structure is disposed on a third perforated material having a first surface and an opposite second surface and wherein the third material is disposed on the first surface and/or the second surface of the third perforated material. It is understood that in such exemplary and unlimiting aspects, the first perforated material, the second perforated material and/or the third perforated material are the same or different.

Also disclosed are methods comprising disposing a first auxiliary layer at at least a portion of the first surface of the first material. For example, in some methods, a first auxiliary is disposed at at least a portion of the second surface of the first material. Yet, in other methods, the first auxiliary layer present on the second surface of the first material is the same or different as the first auxiliary layer present on the first surface of the first material.

In addition or in the alternative disclosed are methods comprising a second auxiliary layer at at least a portion of the first surface of the second material. In some aspects, the methods disclosed herein comprise disposing a second auxiliary layer at at least a portion of the second surface of the second material. In some exemplary and unlimiting methods, the second auxiliary layer present on the second surface of the second material is the same or different as the second auxiliary layer present on the first surface of the second material.

Also disclosed are methods comprising disposing a third auxiliary layer at at least a portion of the first surface of the third material. In some aspects, the methods comprise disposing a third auxiliary layer at at least a portion of the second surface of the third material.

In some methods, the third auxiliary layer present on the second surface of the third material can be the same or different as the third auxiliary layer present on the first surface of the third material. Yet, in still further aspects, each of the first, second or third auxiliary layers can be the same or different.

Also disclosed are methods where the first, second, and/or third perforated material comprises a porous fabric or membrane, wherein the porous fabric or membrane comprises a resorbable material, a non-resorbable material, or a combination thereof. Yet, in other methods, the first, second, and/or third perforated material comprises a porous fabric or membrane, wherein the porous fabric or membrane comprises one or more biocompatible polymers selected from polyethylene, polypropylene, polymethylmethacrylate (PMMA), polystyrene (PS), polytetrafluoroethylene (PTFE), thermoplastic polyurethane (TPU), polyurethane (PU), implantable elastane polymer, polyamide, polyethylene terephthalate (PET), polyethersulfone, poly-lactic-co-glycolic acid (PLGA) or a combination thereof or natural/regenerated fibers selected from cotton, silk, linen, cellulose acetate, collagen, or a combination thereof.

While in other aspects, the first, second, and/or third auxiliary layer configured to impart to at least a portion of the first, second, and/or third material hydrophobic or hydrophilic properties, elastomeric properties, mechanical resilience, adhesive properties, tissue-in-growth inhibition, or any combination thereof.

In certain methods, the first, second, and/or third auxiliary layer comprises a resorbable material, a non-resorbable material, or a combination thereof. Yet, in other methods, the first, second, and/or third auxiliary layer comprises one or more thermoplastic polyurethane (TPU), polyurethane (PU), implantable elastane polymer, or polyethylene, polypropylene, polymethylmethacrylate (PMMA), polystyrene (PS), polytetrafluoroethylene (PTFE), polyamide, polyethylene terephthalate (PET), polyethersulfone, poly-lactic-co-glycolic acid (PLGA).

Also disclosed are aspects directed to the methods comprising disposing the first material between two layers of the first perforated material, and wherein the two layers of the first perforated material are at least partially coupled to each other.

One aspect is directed to the methods comprising the second material disposed between two layers of the second perforated material and wherein the two layers of the second perforated material are at least partially coupled to each other. While another aspect is directed to the methods comprising disposing the third material between two layers of the third perforated material; and wherein at the two layers of the third perforated material are at least partially coupled to each other.

In some methods, at least a portion of the second surface of the first material is disposed on the first surface of the first perforated material. While in other methods, at least a portion of the first surface of the first material is disposed on the second surface of the first perforated material. In still further methods, at least a portion of the second surface of the second material is disposed on the first surface of the second perforated material.

In some aspects disclosed are methods, at least a portion of the first surface of the second material is disposed on the second surface of the second perforated material. In yet other aspects, at least a portion of the second surface of the third material can be disposed on the first surface of the third perforated material.

In still further methods, at least a portion of the first surface of the third material is disposed on the second surface of the third perforated material.

In some methods, at least a portion of the first auxiliary layer and at least a portion of the first perforated material are coupled to each other. In yet further methods, at least a portion of the second auxiliary layer and at least a portion of the second perforated material are coupled to each other. And in still further methods, at least a portion of the third auxiliary layer and at least a portion of the third perforated material are coupled to each other.

Additional aspects of the disclosure will be set forth, in part, in the detailed description, figures, and claims which follow, and in part will be derived from the detailed description or can be learned by practice of the disclosure. It is to be understood that both the foregoing general description and the following detailed description are exemplary and explanatory only and are not restrictive of the disclosure as disclosed.

### BRIEF DESCRIPTION OF THE DRAWINGS

Various aspects are depicted in the accompanying drawings for illustrative purposes and should in no way be interpreted as limiting the scope of the disclosures. In addition, various features of different disclosed aspects can be combined to form additional aspects, which are part of this disclosure. Throughout the drawings, reference numbers may be reused to indicate correspondence between reference elements. However, it should be understood that the use of similar reference numbers in connection with multiple drawings does not necessarily imply a similarity between respective aspects associated therewith. Furthermore, it should be understood that the features of the respective drawings are not necessarily drawn to scale, and the illustrated sizes thereof are presented for the purpose of illustration of inventive aspects thereof. Generally, certain of the illustrated features may be relatively smaller than as illustrated in some aspects or configurations.
**FIGURE** 1 shows an exemplary frame of an exemplary implantable device in accordance with one or more aspects.
**FIGURE 2** shows an exemplary outer skirt material comprising electrospun fibers according to one or more aspects.
**FIGURES 3A-3C** show various exemplary implantable devices according to one or more aspects.
**FIGURES 4A-4F** show SEM images of silk fibroin nanofibers electrospun at concentrations of 5% **(****FIG. 4A****), 6% (****FIG. 4B****),** 7% **(****FIG. 4C****), 8% (****FIG. 4D****),** 9% **(****FIG. 4E****),** and 10% **(****FIG. 4F****)** in 2,2,2-Trifluoroacetic acid (TFA) solvent (from R. Nawalakhe et al., Journal of Fiber Bioengineering & Informatics, 5:3 (2012) 227-242).
**FIGURE** 5 shows a schematic view of one exemplary electrospinning system according to one or more aspects.
**FIGURE** 6 shows a schematic view of one exemplary electrospinning system according to one or more aspects.
**FIGURES 7A-7B** show: **FIG. 7A** shows a schematic view of one exemplary electrospinning system to form an exemplary leaflet system according to one or more aspects; **FIG. 7B** shows a schematic of forming an exemplary leaflet system according to one or more aspects.
**FIGURE 8** shows a schematic summary of needle-less rotating spinnerets (electrospinning along the red arrow) (H. Niu et al., J. of Nanomaterials, 2012, https://doi.org/10.1155/2012/725950)
**FIGURE 9** shows a schematic summary of stationary needle-less spinnerets (electrospinning along the red arrow) (H. Niu et al., J. of Nanomaterials, 2012, https://doi.org/10.1155/2012/725950)
**FIGURES 10A-10B** show an exemplary rotary jet spinning system for applying a material to an exemplary implant device in accordance with one or more aspects **(****FIG. 10A****)** and a close-up view of a reservoir component of the system shown in **FIG. 10A****,** in accordance with one or more aspects **(****FIG. 10B****).**
**FIGURE 11** shows an exemplary setup for forming an inner and/or an outer skirt in one aspect.
**FIGURES 12A-12C** show exemplary layers of the exemplary fabric: **FIG. 12A** shows an exemplary perforated material; **FIG. 12B** shows an exemplary plurality of electrospun fibers; **FIG. 12C** shows an exemplary auxiliary material in one aspect.
**FIGURES 13A-13C** show exemplary configuration of the materials in various aspects.

### DETAILED DESCRIPTION

The present disclosure can be understood more readily by reference to the following detailed description, examples, drawings, and claims, and their previous and following description. However, before the present articles, systems, and/or methods are disclosed and described, it is to be understood that this disclosure is not limited to the specific or exemplary aspects of articles, systems, and/or methods disclosed unless otherwise specified, as such can, of course, vary. It is also to be understood that the terminology used herein is for the purpose of describing particular aspects only and is not intended to be limiting.

The following description of the disclosure is provided as an enabling teaching of the disclosure in its best, currently known aspect. To this end, those skilled in the relevant art will recognize and appreciate that many changes can be made to the various aspects of the disclosure described herein while still obtaining the beneficial results of the present disclosure. It will also be apparent that some of the desired benefits of the present disclosure can be obtained by selecting some of the features of the present disclosure without utilizing other features. Accordingly, those of ordinary skill in the pertinent art will recognize that many modifications and adaptations to the present disclosure are possible and may even be desirable in certain circumstances and are a part of the present disclosure. Thus, the following description is again provided as illustrative of the principles of the present disclosure and not in limitation thereof.

### DEFINITIONS

**As** used in this application and in the claims, the singular forms "a,"" "an," and "the" include the plural forms unless the context clearly dictates otherwise. Thus, for example, a reference to a "material" includes aspects having two or more such materials unless the context clearly indicates otherwise.

It is also to be understood that the terminology used herein is for the purpose of describing particular aspects only and is not intended to be limiting. As used in the specification and in the claims, the term "comprising" can include the aspects "consisting of" and "consisting essentially of." Additionally, the term "includes" means "comprises."

For the terms "for example," "exemplary," and "such as," and grammatical equivalences thereof, the phrase "and without limitation" is understood to follow unless explicitly stated otherwise.

Similarly, when values are expressed as approximations, by use of the antecedent "about," it will be understood that the particular value forms another aspect. It will be further understood that the endpoints of each of the ranges are significant both in relation to the other endpoint and independently of the other endpoint. Unless stated otherwise, the term "about" means within 5% (e.g., within 2% or 1%) of the particular value modified by the term "about."

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

As used herein, the term "composition" is intended to encompass a product comprising the specified ingredients in the specified amounts, as well as any product which results, directly or indirectly, from a combination of the specified ingredients in the specified amounts.

A weight percent (wt.%) of a component, unless specifically stated to the contrary, is based on the total weight of the formulation or composition in which the component is included. As used herein, the terms "optional" or "optionally" mean that the subsequently described event or circumstance may or may not occur and that the description includes instances where said event or circumstance occurs and instances where it does not.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present. Other words used to describe the relationship between elements or layers should be interpreted in a like fashion (e.g., "between" versus "directly between," "adjacent" versus "directly adjacent," "on" versus "directly on").

As used herein, the term "and/or" includes any and all combinations of one or more of the associated listed items.

It will be understood that, although the terms "first," "second," etc., may be used herein to describe various elements, components, regions, layers and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of example aspects.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein interpreted accordingly.

The terms "fiber" and "material comprising a plurality of fibers" are used herein according to their broad and ordinary meanings and may refer to any type of natural or synthetic substance or material that is significantly longer than it is wide, including any elongate or relatively fine, slender, and/or threadlike piece, filament, cord, yarn, plie, strand, line, string, or portion thereof. Furthermore, "fiber" or "material comprising a plurality of fibers" can refer to a single filament or collectively to a plurality of filaments. Examples of material comprising a plurality of fibers in accordance with aspects of the present disclosure include, but are not limited to, any type of cloth, fabric, or textile. It is understood that in certain unlimiting aspects, the term "material comprising a plurality of fibers" can refer to cloth, fabric, textile, or interlocking-fiber material that can cover or form certain features of the disclosed devices.

As used herein, the term "polyester" refers to a category of polymers that contain the ester functional group in their main chain. Polyesters disclosed herein include naturally occurring chemicals, such as in the cutin of plant cuticles, as well as synthetics produced through step-growth polymerization. In certain examples, the polyesters comprise polyethylene terephthalate (PET) homopolymer and copolymers, polypropylene terephthalate (PPT) homopolymer and copolymers and polybutylene terephthalate (PBT) homopolymer and copolymers, and the like, including those that contain comonomers such as cyclohexane dimethanol, cyclohexane dicarboxylic acid, isophthalic acid, and the like.

The term "polyamide," as utilized herein, is defined to be any long-chain polymer in which the linking functional groups are amide (-CO-NH-) linkages. The term polyamide is further defined to include copolymers, terpolymers, and the like, as well as homopolymers and also includes blends of two or more polyamides. In some aspects, the plurality of polyamide fibers comprise one or more of nylon 6, nylon 66, nylon 10, nylon 612, nylon 12, nylon 11, or any combination thereof. In other aspects, the plurality of polyamide fibers comprise nylon 6 or nylon 66. In yet other aspects, the plurality of polyamide fibers are nylon 6. In a yet further aspect, the plurality of polyamide fibers are nylon 66.

As defined herein, the term "polyolefin" refers to any class of polymers produced from a simple olefin (also called an alkene with the general formula CₙH₂ₙ) as a monomer. In some aspects, the polyolefins include but are not limited to polyethylene, polypropylene, both homopolymer and copolymers, poly(l-butene), poly(3-methyl-l-butene), poly(4- methyl- 1-ρentene) and the like, as well as combinations or mixtures of two or more of the foregoing.

As defined herein, the term "polyurethane" refers to any class of polymers composed of a chain of organic units joined by carbamate (urethane, R₁-O-CO-NR₂-R₃, wherein R1, R2 and R3 are the same or different) links.

As defined herein, the term "polyether" refers to any class of polymers composed of a chain of organic units joined by an ether group.

As defined herein, the term "polyurea" refers to any class of polymers where alternative monomer units of isocyanates and amines react with each other to form urea linkages.

As used herein, the term "substantially" means that the subsequently described event or circumstance completely occurs or that the subsequently described event or circumstance generally, typically, or approximately occurs.

Still further, the term "substantially" can in some aspects refer to at least about 90 %, at least about 91 %, at least about 92 %, at least about 93 %, at least about 94 %, at least about 95 %, at least about 96 %, at least about 97 %, at least about 98 %, at least about 99 %, or about 100 % of the stated property, component, composition, or other condition for which substantially is used to characterize or otherwise quantify an amount.

As used herein, the term "substantially," in, for example, the context "substantially identical" or "substantially similar" refers to a method or a system, or a component that is at least about 90%, at least about 91%, at least about 92%, at least about 93%, at least about 94%, at least about 95%, at least about 96%, at least about 97%, at least about 98%, at least about 99%, or about 100% by similar to the method, system, or the component it is compared to.

Although the operations of exemplary aspects of the disclosed method may be described in a particular, sequential order for convenient presentation, it should be understood that disclosed aspects can encompass an order of operations other than the particular, sequential order disclosed. For example, operations described sequentially may, in some cases, be rearranged or performed concurrently. Further, descriptions and disclosures provided in association with one particular aspect are not limited to that aspect and may be applied to any aspect disclosed.

Moreover, for the sake of simplicity, the attached figures may not show the various ways (readily discernable, based on this disclosure, by one of ordinary skill in the art) in which the disclosed system, method, and apparatus can be used in combination with other systems, methods, and apparatuses. Additionally, the description sometimes uses terms such as "produce" and "provide" to describe the disclosed method. These terms are high-level abstractions of the actual operations that can be performed. The actual operations that correspond to these terms can vary depending on the particular implementation and are, based on this disclosure, readily discernible by one of ordinary skill in the art.

### IMPLANTABLE PROSTHETIC VALVE

Aspects of the technology disclosed herein are directed to implantable prosthetic devices and various components of such devices. More specifically, some of the aspects related to implantable prosthetic valves.

It is understood that commonly many components of the medical devices can be covered, at least partially, with materials comprising a plurality of fibers or fibrous materials. Examples of medical device components that can be covered or otherwise associated with a cloth or other materials comprising fibers include certain stents, which can generally comprise a conduit form configured to be placed in a body to create or maintain a passageway within the body or to provide a relatively stable anchoring structure for supporting one or more other devices or anatomy. At least partially cloth-covered stents can be used for a variety of purposes, such as for expansion of certain vessels, including blood vessels, ducts, or other conduits, whether vascular, coronary, biliary, or other types. In the context of prosthetic heart valve devices, a stent can serve as a structural component for anchoring the prosthetic heart valve to the tissue of a heart valve annulus. Such a stent can have varying shapes and/or diameters.

It should be understood that prosthetic heart valve implants, as well as many other types of prosthetic implant devices and other types of devices, can include various cloth-covered components and/or portions. For example, a sealing portion of a medical implant device, such as a prosthetic heart valve skirt component/portion, can be sutured to a frame thereof to help prevent blood from leaking around the outer edges or circumference of the device.

In some implementations, cloth coverings for medical device components can be secured using sutures. For example, in some implementations, a human operator may handle and execute sutures on implant device components to secure a cloth thereto. However, the execution of sutures by a human operator may be relatively difficult and/or cumbersome in certain situations. For example, where small stitches are to be made with relatively high precision, the complexity and/or associated operator burden may result in injury/strain and/or undesirably-low product quality. Furthermore, medical implant devices, such as certain heart valve implant devices, may require upward of a thousand sutures, or more, which can involve substantially labor-intensive and error-susceptible suturing procedures. Therefore, reducing the collaborative human involvement in the application of fibrous material to medical device components can be desirable to improve quality and efficiency and/or to reduce operator strain.

Certain aspects disclosed herein provide for the implantable prosthetic devices that can comprise materials comprising a plurality of fibers comprising an electrospun silk. Even further disclosed herein are aspects where these materials and devices are formed using electrospinning devices, systems, processes, and mechanisms. Examples of medical implant devices and heart valve structures that may be applicable to certain aspects presented herein are disclosed in WIPO Publication No. WO 2015/070249.

The aspects disclosed below include materials comprising a plurality of fibers, wherein at least one fiber of the plurality of fibers comprises an electrospun silk. Such materials, as disclosed in detail below, can be formed by electrospinning processes. Electrospinning processes generally employ high voltages to create an electric field between a droplet of polymer solution at the tip of a needle and a collector plate, as described in detail below. In certain aspects, one electrode of the voltage source can be placed into the solution, and the other is connected to the collector. This creates an electrostatic force. As the voltage is increased, the electric field intensifies, causing a force to build-up on the pendant drop of polymer solution at the tip of the needle. This force acts in a direction opposing the surface tension of the drop. The increasing electrostatic force causes the drop to elongate, forming a conical shape. When the electrostatic force overcomes the surface tension of the drop, a charged, continuous jet of a solution is ejected from the cone. The jet of solution accelerates towards the collector, whipping and bending wildly. As the solution moves away from the needle and toward the collector, the jet rapidly thins and dries as the solvent evaporates. On the surface of the grounded collector, a nonwoven mat of randomly oriented solid nanofibers is deposited. Certain methods, devices, and systems relating to electrospinning concepts that may be applicable to aspects of the present disclosure are disclosed in U.S. Publication No. 2017/0325976. The specific aspects of the methods are also disclosed below in more detail.

The aspects disclosed herein are related to materials comprising a plurality of electrospun silk fibers. To electrospun fibers, often and as described in detail below, a natural protein, silk fibroin (SF), can be utilized. SF derived from silkworm plays a crucial role in biomedical applications and tissue engineering (W.H. Zhou et al., ACS Appl. Mater. Inter. 9 (2017), 25830-25846; J. Du et al., App. Surf. Sci., 447 (2018), 269-278). Silkworm is mainly composed of silk fibroin coated with sericin, and their content is over 95%. It was found that there is a small amount of carbohydrates and other impurities in silkworms can be present. SF structure is mainly composed of glycine (46%), alanine (29%), serine (18%), and other 18 kinds of amino acids (J. Brown et al., Acta Biomater. 11 (2015), 212-221). SF consists of a light (L) chain polypeptide and a heavy (H) chain polypeptide linked together via a single disulfide bond at the C-terminus of the H-chain, forming an H-L complex (I.D. Koh et al., Prog. Polym. Sci. 46 (2015), 86-110). SF is considered to be one of the biological materials with the most applicative prospect due to the unique properties of excellent biocompatibility (Y.F. Feng et al., ACS Sustain. Chem. Eng. 5 (2017), 6227-6236), control of excellent mechanical properties (F. Teule et al., Proc. Natl. Acad. Sci. U.S.A. 109 (1012) 923-928), biodegradability (A. Teimouri et al., Polym. Degrad. Stabil. 121 (2015), 18-29; F. M. Miroiu et al., Appl. Surf. Sci. 355 (2015), 1123-1131) hemocompatibility, cytocompatibility and its interactions with cells.

In the aspects disclosed herein, silk fibroin was found to be a good alternative to the previously disclosed materials and textiles used in the implantable prosthetic devices. It was shown that the use of the silk fibroin as a source of the fibrous material allows controlling the rate of degradation of the formed components as an important feature of function tissue design. Without wishing to be bound by any theory, it was assumed that the use of the silk-fibroin as a source for electrospun silk allows successfully matching the rate of scaffold degradation to the rate of tissue growth.

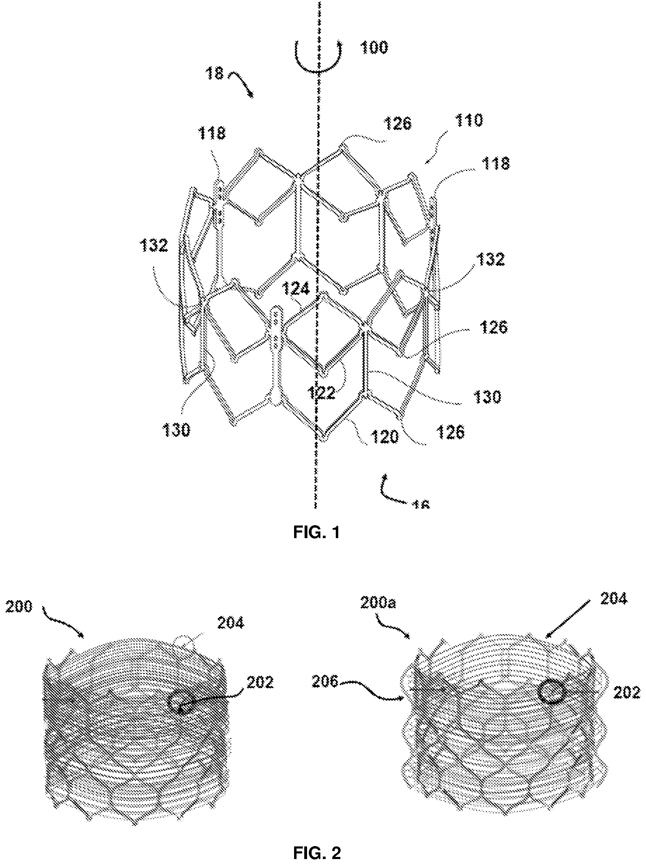

**FIG. 1** illustrates an exemplary frame **110** having a central longitudinal axis **100** extending from the inflow portion **16** to outflow portion **18** of an example stent that can be used in an implantable prosthetic device in accordance with one or more aspects of the present disclosure. The frame **110** may be made from laser-cut tubing of a plastically-expandable metal or other at least partially rigid material. It is understood that since the implantable prosthetic valve is radially collapsible to a collapsed configuration and radially expandable to an expanded configuration, frame **110** can be also at least partially radially collapsible to a collapsed configuration and at least partially radially expandable to an expanded configuration. Although a laser-cut stent is shown, it should be understood that other frames can be used in the devices and methods disclosed herein, including stents comprising rigid rings, spirally-wound tubes, and other tubes/conduits that fit within, for example, a heart valve annulus and that define an orifice therethrough for the passage of blood.

The frame **110** can be at least partially self-expanding and/or maybe mechanically expandable (*e.g*., balloon-expandable). For example, a self-expanding frame of the stent can be crimped or otherwise compressed into a small tube and may possess sufficient elasticity to spring outward by itself when a restraint, such as an outer sheath/catheter, is removed. In contrast, a balloon-expanding stent may comprise a material that is relatively less elastic and is capable of plastic expansion from the inside-out when converting the stent from a contracted diameter/configuration to an expanded diameter/configuration. The plastic expansion may be accomplished with a balloon or other device, such as a device with mechanical fingers. With such balloon-expanding stents, the stent frame may plastically deform after the application of a deformation force, such as an inflating balloon or expanding mechanical fingers.

The frame **110** of a stent (*e.g*., self-expanding stent or balloon-expanding stent) may be used as part of a prosthetic heart valve having single-stage implantation in which a surgeon secures a heart valve having a fibrous anchoring skirt and valve member to a heart valve annulus as one unit or piece. Certain stent solutions for aortic valve replacement in accordance with some aspects of the present disclosure are disclosed in U.S. Patent No. 8,641,757**.** In some implementations, an exemplary delivery system advances the valve implant device with the stent at the leading or distal end until it is located within the valve annulus and/or left ventricular outflow tract, at which point a balloon can inflate to expand the stent against the aortic annulus and/or ventricular tissue.

As shown in **FIG. 1****,** the stent frame **110** is generally annular and/or cylindrical and includes a plurality of angularly-spaced, vertically-extending, commissure attachment posts, or struts, **118.** Posts **118** can be interconnected at least by a lower row of circumferentially-extending struts **120** and one or more upper rows of circumferentially extending struts **122** and **124,** respectively. The struts in each row can be arranged in a zig-zag or generally saw-tooth-like pattern extending in the direction of the circumference of the frame, as shown. Adjacent struts in the same row can be interconnected to one another to form an angle between about 90-110 degrees. The angle between adjacent struts can be selected to optimize the radial strength of the frame 110 when expanded yet still permit the frame **110** to be evenly crimped and expanded.

In the illustrated aspect, pairs of adjacent circumferential struts in the same row are connected to each other by a respective, generally U-shaped crown structure or portion **126.** The crown structures **126** can each include a horizontal portion extending between and connecting the adjacent ends of the struts such that a gap is defined between the adjacent ends and the crown structure connects the adjacent ends at a location offset from the strut's natural point of intersection. The crown structures **126** can significantly reduce residual strains on the frame **110** at the location of the struts **120, 122, 124** during crimping and expanding of the frame **110.** Each pair of struts **122** connected at a common crown structure **126** can generally form a cell with an adjacent pair of struts **124** in the row above. Each cell can be connected to an adjacent cell at node **132.** Each node **132** can be interconnected with the lower row of struts by a respective vertical (axial) strut **130** that is connected to and extends between a respective node **132** and a location on the lower row of struts **120** where two struts are connected at their ends opposite of a crown structure **126.**

In certain aspects, lower struts **120** have a greater thickness or diameter than upper struts **122, 124.** In one implementation, for example, lower struts **120** have a thickness of about 0.42 mm and upper struts **122, 124** have a thickness of about 0.38 mm. In the particular aspect of **FIG. 1****,** because there is only one row of lower struts **120** and two rows of upper struts **122, 124,** enlargement of the lower struts 120 with respect to the upper struts **122, 124** can advantageously enhance the radial strength of the frame **110** at the lower area of the frame and/or allow for more uniform expansion of the frame. Columns of frame **110** can be defined by the adjoining pairs of struts **120, 122, 124** extending between two axially-extending struts **130.** In some aspects, frame **110** comprises three 120-degree segments, with each segment being bounded by two posts **118.** Accordingly, frame **110** of the particular aspect of **FIG. 1** includes 9 total columns. In some aspects, the number of columns and rows may be desirably minimized to reduce the overall crimp profile of the frame **110** and/or associated valve. Again, it is understood that this frame is only exemplary, and other frames can be used in the disclosed implantable prosthetic devices and methods of making the same.

In still further aspects, the implantable device as disclosed herein comprises a leaflet structure positioned with the frame, and/or an inner skirt positioned along the inner surface of the frame and/or at least one outer skirt positioned around the outer surface of the frame. As disclosed herein, at least a portion of one of the leaflet structure, the inner skirt, or the at least one outer skirt comprises a material comprising a plurality of fibers, wherein at least one fiber of the plurality of fibers comprises electrospun silk. However, it is further understood that in certain aspects, any or all of the disclosed components can comprise the material comprising the plurality of electrospun silk fibers,

In certain aspects, when at least a portion, or a whole surface, of the inner skirt comprises the material comprising the plurality of fibers comprising the electrospun silk, the material is referred to as a first material. While in other aspects, when at least a portion, or a whole surface, of the outer skirt comprises the material comprising the plurality of fibers comprising the electrospun silk, the material is referred to as a second material. While in still further aspects, when at least a portion, or a whole surface, of the leaflet structure comprises the material comprising the plurality of fibers comprising the electrospun silk, the material is referred to as a third material. It is understood that these definitions are used to distinguish between the materials of each component and do not indicate a specific order or specific material. It is understood, as described below, that the first material, the second material, and/or the third material can be the same or different.

It is further understood, and as it is described below, each or all of the materials comprising electrospun silk can be engineered to have specific properties depending on the desired application. For example, a fibrous material (the third material as disclosed herein) used to form the leaflet structure is designed to exhibit properties that are different from the properties required for the material used for the inner (the first material) or outer skirt (the second material) of the implantable device. Therefore, described herein are the aspects where the first, second, and third materials are the same as well the aspects where the first, second, and third materials are different.

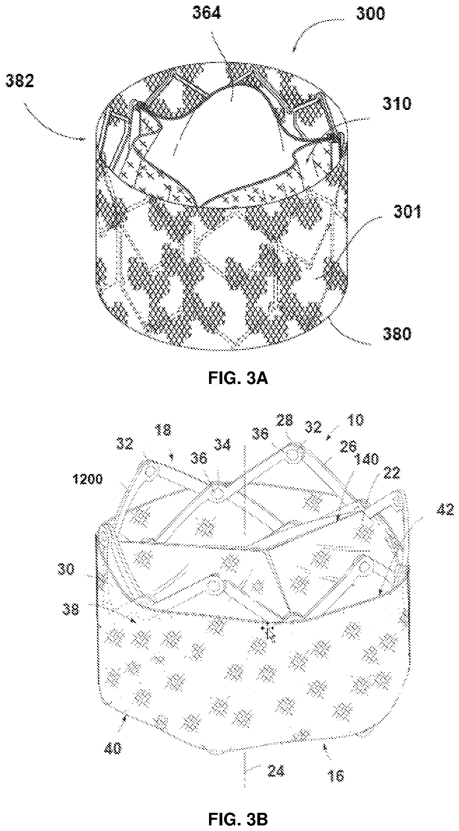

Exemplary and unlimiting implantable prosthetic devices having various components are shown in **FIGS. 3A-3B****.** For example, **FIG. 3A** illustrates a device **300** having a frame **310** that is analogous to the frame **210 described in** **FIG. 1****. As assembled,** the valve **300** in the illustrated aspect includes a leaflet structure **364** supported by the stent frame **310,** which includes an inner skirt **301** applied to the stent frame **310.** In this illustrated aspect, either or both of the leaflet structure **364** and the inner skirt **301** can comprise a material comprising a plurality of fibers, wherein at least one fiber of the plurality of fibers comprises electrospun silk. In such aspects, for example, the leaflet structure can comprise the third material as disclosed herein, while the inner skirt can comprise the first material as also disclosed herein. However, it is also understood that disclosed herein are the aspects where, for example, only the inner skirt comprises the first material, and the leaflet structure does not comprise the third material as described. In such exemplary and unlimiting aspects, the leaflet structure can comprise any other commonly used for the desired application materials. In still further exemplary aspects, only the leaflet structure comprises the third material as disclosed herein, and the inner structure does not comprise the first material as described. In such exemplary and unlimiting aspects, the inner skirt can comprise any other commonly used for the desired application materials. In still further aspects, these materials can exhibit any of the disclosed herein properties.

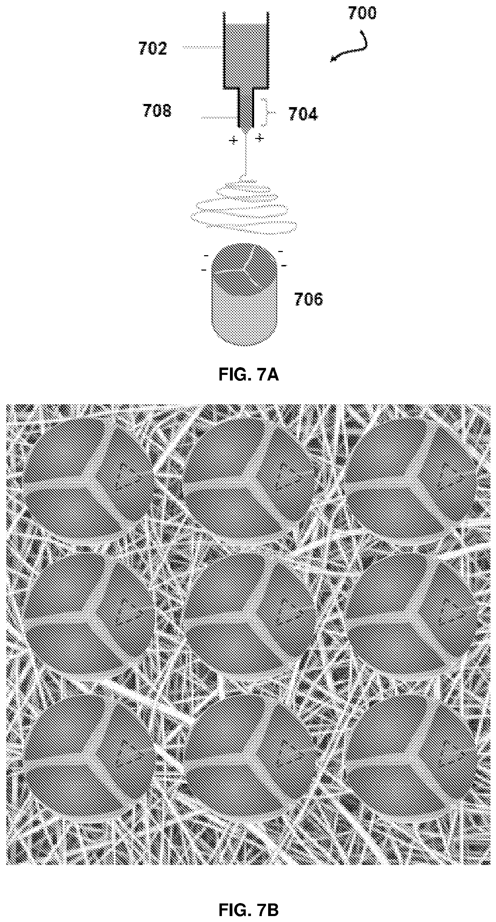

In still further aspects, and as discussed in detail below, the leaflet structure **364** can be formed by the electrospinning methods. In certain aspects, the third material comprising the leaflet structure can be formed by electrospinning of the silk fibroin fibers, for example, on the third predetermined mandrel. In such aspects, the third predetermined mandrel can have a form of the desired leaflet structure (for example, **FIG. 7A****),** or it can be cut to the desired form from the sheet of the third material prepared by the electrospinning (for example, **FIG. 7B****).**

Similarly, the inner skirt **301** can be formed by direct electrospinning as discussed in detail below, or it can be prepared from the sheet of the first material, cut to the desired form, and then, is attached to the frame by any known in the art methods. For example, it can be attached to the inner surface of the frame with a fastener. In certain aspects, the fastener can comprise glue and/or sutures.

The valve implant device **300** can be suitable for implantation in the annulus of a native aortic valve, for example, but also can be adapted to be implanted in other native valve annuluses of the heart or various other ducts or orifices of the body. The valve implant device **300** has an inflow end **380** and an outflow end **382.**

The valve implant device **300** and stent frame **310** as disclosed herein are configured to be radially collapsible to a collapsed or crimped state for introduction into the body within a delivery catheter and radially expandable to an expanded state for implanting the valve **300** at a desired location in the body (*e.g*., the native aortic valve). For example, and without limitation, the stent frame **310** can be made of a plastically-expandable material that permits crimping of the valve to a smaller profile for delivery and expansion of the valve using an expansion device, such as the balloon of a balloon catheter. Alternatively, the valve implant device **300** can be a self-expanding valve, wherein the frame is made of a self-expanding material such as memory metal (*e.g.*, Nitinol). A self-expanding valve can be crimped to a smaller profile and held in the crimped state with a restraining device, such as a sheath covering the valve. When the valve is positioned at or near the target site, the restraining device may be removed to allow the valve to self-expand to its expanded, functional size. It is further understood, however, that other suitable for this purpose materials can also be employed to form the frame.

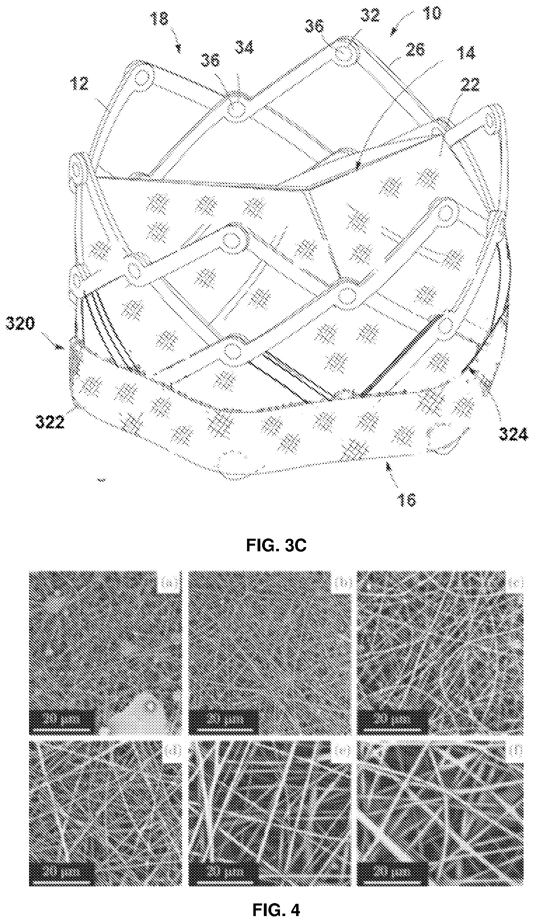

Other exemplary aspects of implantable medical devices are shown in **FIGS. 3B** **and** **3C****.** For example, and without limitation, **FIG. 3B** illustrates an exemplary aspect of a radially collapsible and expandable prosthetic valve **10** shown in its deployed, expanded configuration. The prosthetic valve can include an annular stent or frame **1200** and a leaflet structure **140** situated within and coupled to the frame **1200.** Frame **1200** can have an inflow end portion **16** and an outflow end portion **18.** The leaflet structure can comprise a plurality of leaflets **22.** In certain aspects, the leaflet structure can comprise three leaflets. In such exemplary aspects, such three leaflets can be arranged to collapse in a tricuspid arrangement similar to the aortic valve. Alternatively, the prosthetic valve can include two leaflets **22** configured to collapse in a bicuspid arrangement similar to the mitral valve, or more than three leaflets, depending upon the particular application. It is understood that disclosed herein are the aspects where the leaflet structure **140** comprises the third material as disclosed herein. However, also disclosed herein are the aspects where the leaflet structure does not comprise the third material as disclosed herein. In such exemplary aspects, the leaflet structure can comprise any known in the art materials. The prosthetic valve **10** can define a longitudinal axis **24** extending through the inflow end portion **16** and the outflow end portion **18.**

Frame **1200** can be made from any of various biocompatible materials, such as stainless steel or a nickel-titanium alloy ("NiTi"), for example, Nitinol. With reference to **FIG. 3B****,** frame **1200** can include a plurality of interconnected lattice struts **26** arranged in a lattice-type pattern and forming a plurality of apices **28** at the outflow end **18** of the prosthetic valve. The struts **26** can also form similar apices at the inflow end **16** of the prosthetic valve (which are covered by an outer skirt **30** described in greater detail below). The lattice struts **26** are shown positioned diagonally or offset at an angle relative to, and radially offset from, the longitudinal axis **24** of the prosthetic valve. Again, it is understood that the configuration depicted in **FIG. 3A** is exemplary only, and in other aspects, the lattice struts **26** can be offset by a different amount than depicted in **FIG. 3A****,** or some or all of the lattice struts **26** can be positioned parallel to the longitudinal axis of the prosthetic valve.

The lattice struts **26** can be pivotably coupled to one another. In the illustrated aspect, for example, the end portions of the struts **26** forming the apices **28** at the outflow end **18** and at the inflow end **16** of the frame can have a respective opening **32.** The struts **26** also can be formed with apertures **34** located between the opposite ends of the struts. Respective hinges can be formed at the apices **28** and at the locations where struts **26** overlap each other between the ends of the frame via fasteners **36,** which can comprise rivets or pins that extend through the apertures **32, 34.** The hinges can allow the struts **26** to pivot relative to one another as frame **1200** is expanded or contracted, such as during assembly, preparation, or implantation of the prosthetic valve **10.** For example, frame **1200** (and, thus, the prosthetic valve **10)** can be manipulated into a radially compressed or contracted configuration, coupled to a delivery apparatus, and inserted into a subject for implantation. Once inside the body, the prosthetic valve **10** can be manipulated into an expanded state and then released from the delivery apparatus. Additional details regarding frame **1200,** the delivery apparatus, and devices and techniques for radially expanding and collapsing the frame can be found in U.S. Publication No. 2018/0153689. Additional details about such an exemplary prosthetic valve can also be found in U.S. Publication No. 2019/0046314.

As further illustrated in **FIG. 3A****,** the prosthetic valve **10** can include a sealing element configured as an outer skirt **30.** The outer skirt **30,** as shown herein, can comprise the second material as disclosed herein. While in other aspects, the outer skirt **30** can also comprise any other known in the art materials that can be suited for the desired application. In such aspects, another skirt (not shown) known as an inner skirt can be attached to the frame underneath of the outer skirt **30.** In the aspects where also the inner skirt is present, the inner skirt can comprise the first material as disclosed herein. While in other aspects, when the inner skirt is present, the inner skirt can also comprise other materials known in the art and applicable for the desired application.

The outer skirt **30** can be configured to establish a seal with the native tissue at the treatment site to reduce or prevent paravalvular leakage. The outer skirt **30** can include a main body portion **38** disposed about an outer circumference of the frame **1200.** The outer skirt **30** can be secured to the frame by direct electrospinning, as discussed in detail below and show, for example, in **FIG. 2****,** or it can be formed by a separate electrospinning process and attached to the frame with a fastener. In such exemplary aspects, if the outer skirt **30** is formed by indirect electrospinning, the desired configuration of the skirt can be cut out to the desired shape and dimension and attached to the frame. Any fasteners or joining techniques known in the art can be used for this purpose. For example, it can be attached with glue, or it can be attached with the sutures, or it can be ultrasonically welded. Further details regarding transcatheter prosthetic heart valves, including the manner in which the leaflets **22** can be coupled to frame **1200,** can be found, for example, in U.S. Pat. Nos. 6,730,118, 7,393,360, 7,510,575, 7,993,394, and

**FIG. 3C** illustrates a prosthetic valve **10,** including another aspect of a sealing member or an outer skirt **320.** In the illustrated and unlimiting aspect, skirt **320** comprises the second material as described herein and configured as a fabric strip **322** having an edge portion **328.** Such outer skirt **320** can be formed by either direct electrospinning, as discussed below, or can be cut from the third material prepared as a sheet to the desired form. In certain exemplary aspects, skirt **320** can be secured to struts **26** to form layers of the skirts (not shown). The securing of the struts can be done again by direct electrospinning of the silk fibers or attaching the skirt materials prepared separately with fasteners such as glue or various sutures. It is further understood that any configurations of the skirt coupling with the frame can be used depending on the desired application. It is understood that the disclosed above implantable prosthetic devices are exemplary only, and other devices can be formed. Some additional examples of other prosthetic spacer devices are described further in U.S. Patent Publication Number 2018/0325661, U.S. Patent Publication Number 2017/0325976, and U.S. Patent Application No.

Also disclosed herein are the aspects where each fiber of the plurality of fibers present in the first material and/or the second material and/or third material have first extending direction and a plurality of undulation. In yet further aspects, the first extending direction can comprise a circumferential direction, a radial direction, or a combination thereof. While in other aspects, the plurality of undulations are present in the collapsed configuration. In still further aspects, the plurality of undulations are configured to straighten when the implantable prosthetic valve is in the expanded configuration. Such exemplary aspects are shown in **FIG. 2. FIG. 2** shows exemplary devices **200** (in the expanded configuration) and **200a** (in the collapsed configuration). In this exemplary aspect, an exemplary outer skirt **204** is formed by direct electrospinning on the frame **202.** Exemplary undulations **206** can be observed in **FIG. 2****.** It is understood that these undulations can be formed by any methods known in the art. In certain and unlimiting aspects, the undulations can be formed by electrospinning the second material on a fully expanded frame **202** and then collapsing the frame to the collapsed position. In still further aspects, the undulations can be formed by, for example, and without limitation, introducing some extension means during electrospinning and removing this means when the electrospinning of the desired material is complete.

As disclosed above, in certain aspects, to improve adhesion between the frame and the components electrospun on or attached thereto, an adhesive material can be disposed between at least a portion of the annular frame and at least a portion of the outer skirt and/or between at least a portion of the annular frame and at least a portion of the inner skirt. As in any of the disclosed above aspects, at least a portion of the inner skirt can be attached to the annular frame by direct electrospinning of the plurality of fibers on at least a portion of the inner surface of the annular frame. While in other aspects, the inner skirt material can be formed by the electrospinning from silk fibroin solution to form a sheet of the material comprising electrospun silk from which the inner skirt can be cut out by any known in the art methods (for example, such as laser cutting or ultrasonic cutting) and attached to the frame. Similarly, there are also aspects where at least a portion of the outer skirt is attached to at least a portion of the annular frame by direct electrospinning of the plurality of fibers on at least a portion of the outer surface of the annular frame. While in other aspects, the outer skirt material can also be formed by the electrospinning from silk fibroin solution to form a sheet of the material comprising electrospun silk from which the outer skirt can be cut out by any known in the art methods (for example, such as laser cutting or ultrasonic cutting) and attached to the frame.

In yet further aspects, at least a portion of the plurality of fibers has a random orientation. While in other aspects, at least a portion of the plurality of fibers has a predetermined aligned orientation. In yet further aspects, the first and/or the second and the third/material can comprise at least a portion of the plurality of fibers have a random orientation, and at least a portion of the plurality of fibers have a predetermined aligned orientation. In such exemplary aspects, the orientation of the fibers or the lack thereof can be controlled by various parameters of the electrospinning procedures, as disclosed below.

In certain aspects, to control and/or alter mechanical properties of the first and/or the second and/or the third material, additional fibers can be present in these materials. For example, the plurality of fibers can comprise a resorbable material, a non-resorbable material, or a combination thereof. For example, in certain aspects, to achieve desired mechanical properties, the plurality of fibers can further comprise thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof. In certain aspects, where only bioresorbable and biocompatible fibers are desired, the plurality of fibers can comprise polyglycolic acid (PGA), poly(ester urethane)urea, polylactic acid (PLA), polycaprolactone (PCL), poly(lactic-co-glycolic acid) (PLGA) or any combination thereof. In aspects where the final component is not desired to be bioresorbable, other fibers can be present. For example, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), or a combination thereof. It is understood that any know variation of the PET can be used, such for example, and without limitation, high tenacity PET can also be utilized.

In certain exemplary and unlimiting aspects, and depending on the desired applications, at least a portion of the plurality of fibers can comprise a bicomponent fiber. It is understood that any known in the art configurations of the bicomponent fibers can be utilized. For example, and without limitation, the bicomponent fiber can comprise a side-by-side configuration, a sheath-core configuration, an islands-in-the-sea configuration, a tri-lobal, a segmented pie configuration, or any combination thereof. In still further exemplary aspects, the bicomponent fiber comprises the sheath-core configuration. In some aspects, a sheath and/or core can comprise a resorbable material, a non-resorbable material, or a combination thereof. In certain aspects, a sheath of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and wherein a core of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof. While is still further exemplary aspects, a sheath of the bicomponent fiber can comprise silk, while a core of the bicomponent fiber can comprise one or more of thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof.

In still further aspects, the plurality of fibers can have an average diameter from about 3 nm to about 15,000 nm, including exemplary values of about 5 nm, about 10 nm, about 15 nm, about 20 nm, about 25 nm, about 30 nm, about 35 nm, about 40 nm, about 45 nm, about 50 nm, about 55 nm, about 60 nm, about 65 nm, about 70 nm, about 75 nm, about 80 nm, about 85 nm, about 90 nm, about 95 nm, about 100 nm, about 150 nm, about 200 nm, about 300 nm, about 400 nm, about 500 nm, about 600 nm, about 700 nm, about 800 nm, about 900 nm, about 1,000 nm about 1 ,200 nm, about 1,500 nm, about 2,000 nm, about 2,500 nm, about 3,000 nm, about 3,500 nm, about 4,000 nm, about 4,500 nm, about 5,000 nm, about 5,500 nm, about 6,000 nm, about 6,500 nm, about 7,000 nm, about 7,500 nm, about 8,000 nm, about 8,500 nm, about 9,000 nm, about 8,500 nm, about 10,000 nm, about 10,500 nm, about 11,000 nm, about 11,500 nm, about 12,000 nm, about 12,500 nm, about 12,000 nm, about 12,500 nm, about 13,000 nm, about 13,500 nm, about 14,000 nm, and about 13,400 nm. It is understood that the plurality of fibers can have an average diameter having values between any two foregoing values. It is further understood that the average diameter of the fiber can be controlled by electrospinning parameters, as discussed in detail below.

In still further aspects, wherein the first material, and/or the second material, and/or the third material can have a thickness from about 0.1 µm to about 2 mm, including exemplary values of about 0.2 µm, about 0.5 µm, about 1 µm, 5 µm, about 10 µm, about 15 µm, about 20 µm, about 30 µm, about 50 µm, about 100 µm, about 200 µm, about 300 µm, about 400 µm, about 500 µm, about 600 µm, about 700 µm, about 800 µm, about 900 µm, about 1 mm, about 1.2 mm, about 1.3 mm, about 1.4 mm, about 1. 5 µm, about 1.6 mm, about 1.7 mm, about 1.7 mm, and about 1.9 mm. Still further, the thickness can be any thickness between any two foregoing values. Again, it is further understood that the thickness of the material can be controlled by varying the parameters of the electrospinning process.

In still further aspects, at least a portion of the first material, and/or the second material, and/or third material exhibits porosity. It is understood that, as referenced herein, the term "pore size" refers to the mean size of the nanofiber pores. As used herein, porosity is determined by a ratio of pores to a unit of volume. Again, it is understood that the level of porosity and/or pore size can be controlled by varying the parameters of the electrospinning process. In exemplary aspects disclosed herein, the at least a portion of the first material, and/or the second material, and/or third material can have an average pore size from about 100 nm to about 100 µm, including exemplary values of about 150 nm, about 200 nm, about 300 nm, about 400 nm, about 500 nm, about 600 nm, about 700 nm, about 800 nm, about 900 nm, about 1,000 nm about 1,200 nm, about 1,500 nm, about 2,000 nm, about 2,500 nm, about 3,000 nm, about 3,500 nm, about 4,000 nm, about 4,500 nm, about 5,000 nm, about 5,500 nm, about 6,000 nm, about 6,500 nm, about 7,000 nm, about 7,500 nm, about 8,000 nm, about 8,500 nm, about 9,000 nm, about 9,500 nm, about 1 µm, about 1.5 µm, about 2 µm, about 2.5 µm, about 3 µm, about 3.5 µm, about 4 µm, about 4.5 µm, about 5 µm, about 10 µm, about 15 µm, about 20 µm, about 30 µm, about 40 µm, about 50 µm, about 60 µm, about 70 µm, about 80 µm, and about 90 µm. Still further, the porosity can have any value between any two foregoing values.

Exemplary materials having various average fiber diameter and porosity are shown in **FIG. 4****.** It can be seen that in these exemplary aspects, the fiber diameter and the porosity can be controlled with the concentration of the active material in the electrospinning solution.

Also disclosed herein are aspects where the first material and/or the second material, and/or third material comprise a plurality of layers, wherein each of the plurality of layers comprises electrospun silk, and wherein each of the plurality of layers is disposed on each other. It is understood that the number of layers can be any number that provides for the desired material. In such exemplary aspects, at least a first portion of the plurality of layers can have a surface area that is substantially smaller than a surface area of a second portion of the plurality of layers' surface area. It is understood that such different portions of the layers can be formed by varying electrospinning parameters during the electrospinning process. The electrospinning parameters can include the distance between the spinneret and the collection substrate, the amount of the voltage applied used during electrospinning, extrusion rate, a spinning rate of the collection substrate if it rotates, and the like. For example, some portions of the materials can be more porous than others and thus have a higher surface area than other portions of the materials. In certain exemplary and unlimiting aspects, if substantially no tissue growth is desired, the portions of some of the layers can be constructed to be very dense, less porous, and provide a substantially smooth surface. While in other aspects, where extensive tissue growth is desired, the portions of the layers can have higher porosity and less dense. It is understood again that pore size and, therefore, a surface area can be regulated by varying various electrospinning process parameters.

Also, as disclosed above, various components of the implantable device can have different desired properties. For example, the inner skirt or the leaflet structure of the device may not need to be susceptible to excessive growth or to be rapidly bioresorbable. In such aspects, denser, less porous materials can be utilized. Also, in such aspects, any of the additional fibers, as disclosed above, can be present in the plurality of fibers to increase the mechanical strengths of the materials and regulate bioresorbability and/or biodegradation as desired.

Also disclosed herein are aspects where the first material and/or the second material and/or the third material can comprise various layers comprising a various plurality of fibers. For example, and without limitation, in certain aspects, any of the disclosed materials can have a plurality of layers having different fiber compositions. In some exemplary and unlimiting aspects, the material can have a plurality of layers comprising electrospun silk followed by a plurality of layer comprising electrospun silk and any of the disclosed above polymers, followed by a plurality of layers comprising any of the disclosed above electrospun polymers without the presence of the electrospun silk and so on. Again, it is understood that the described sequence of the plurality of layers is only exemplary, and any or none of the disclosed layers can be present.

For example, at least a portion of the inner skirt further can comprise a first perforated material having a first surface facing the annular frame and an opposite second surface and wherein the first material comprising the plurality of fibers comprising the electrospun silk is disposed on the first surface and/or the second surface of the first perforated material. Yet in another example, at least a portion of the outer skirt further can comprise a second perforated material having a first surface facing the annular frame and an opposite second surface and wherein the second material comprising the plurality of fibers comprising the electrospun silk is disposed on the first surface and/or the second surface of the second perforated material. Yet still, in another example, at least a portion of the leaflet structure can comprise a third perforated material having a first surface facing the annular frame and an opposite second surface and wherein the third material comprising the plurality of fibers comprising the electrospun silk is disposed on the first surface and/or the second surface of the third perforated material.

Also disclosed are examples where at least a portion of the first surface of the first material comprises a first auxiliary layer and/or wherein at least a portion of the second surface of the first material comprises a first auxiliary layer. In such exemplary aspects, the first auxiliary layer present on the second surface of the first material is the same or different as the first auxiliary layer present on the first surface of the first material.

Also disclosed are examples where at least a portion of the first surface of the second material comprises a second auxiliary layer and/or wherein at least a portion of the second surface of the second material comprises a second auxiliary layer. In such exemplary aspects, the second auxiliary layer present on the second surface of the second material is the same or different as the second auxiliary layer present on the first surface of the second material.

Still further are also disclosed examples where at least a portion of the first surface of the third material comprises a third auxiliary layer and/or wherein at least a portion of the second surface of the third material comprises a third auxiliary layer. In such exemplary and unlimiting aspects, the third auxiliary layer present on the second surface of the third material is the same or different as the third auxiliary layer present on the first surface of the third material.

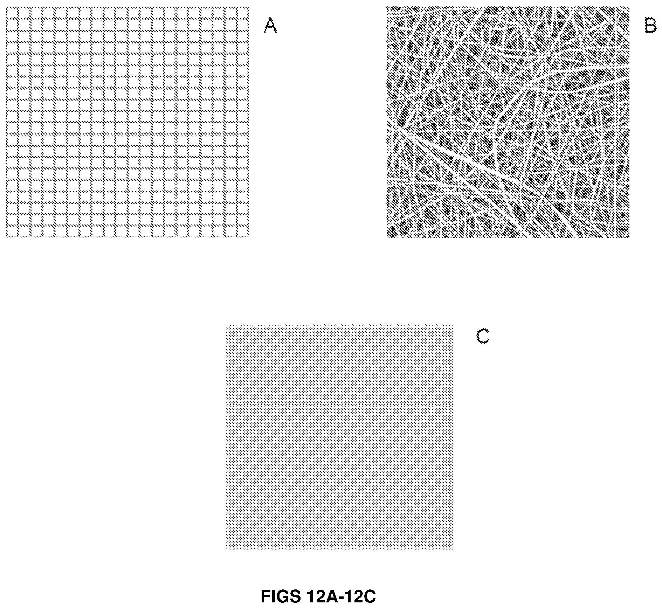

In still further aspects and as shown in **FIGs. 12A-13C****,** the first, second and/or third materials can have various configurations. For example, and without limitations, each or any of these materials can comprise at least one perforated material A **(****FIG. 12A****)** and at least one layer of the disclosed herein electrospun fibers B **(****FIG. 12B****).** While in other aspects, each or any of these materials can comprise the at least one layer of the disclosed herein electrospun fibers B and at least one layer of an auxiliary layer C **(****FIG. 12C****).** While in still other aspects, each or any of these materials can comprise the at least one perforated material A, the at least one layer of the disclosed herein electrospun fibers B and the at least one layer of an auxiliary layer C.

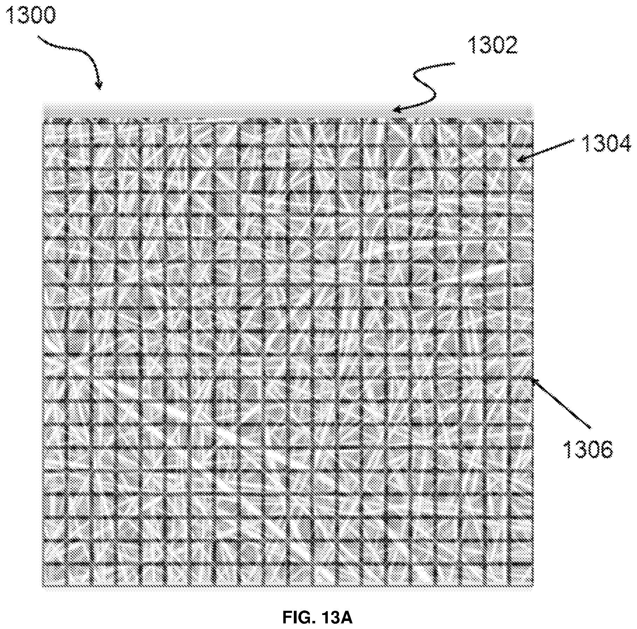

Some exemplary and unlimiting configurations are further shown in **FIG. 13A-****13C.** For example, **FIG. 13A** shows a material configuration **1300** where an inner surface of the at least one layer of the disclosed herein electrospun fibers **1304** is coated or otherwise covered with at least one auxiliary layer **1302,** while a perforated material **1306** is disposed on an outer surface of the at least one layer of the disclosed herein electrospun fibers **1304.** It is understood that the aspects where the auxiliary layer is disposed on the inner surface of the electrospun fibers and the perforated material disposed on the outer surface of the electrospun fibers are also disclosed herein.

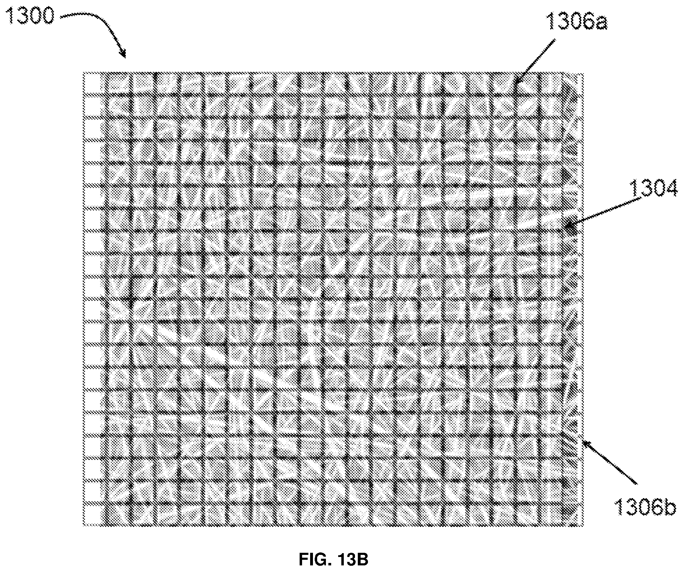

In still further aspects, additional configurations can be considered. For example, as shown in **FIG. 13B****,** the at least one layer of any of the disclosed herein electrospun fibers can be sandwiched between at least two perforated materials **1306a** and **1306b.** It is understood that these two perforated materials can be the same or different and can comprise any of the disclosed below materials.

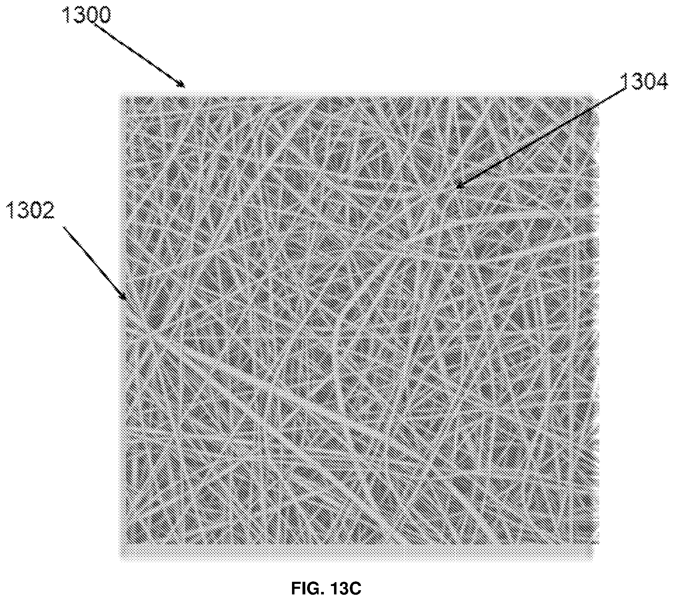

In still further aspects and as shown in **FIG. 13C****,** the material configuration can be free of the perforated material but comprise at least one auxiliary layer. In such aspects, any of the listed below auxiliary layers can be utilized.

In some aspects, any of the perforated materials can comprise a porous fabric or membrane, wherein the porous fabric or membrane comprises one or more biocompatible polymers comprising resorbable or non-resorbable materials, or a combination thereof. In still further aspects, the biocompatible polymers can be selected from polyethylene, polypropylene, polymethylmethacrylate (PMMA), polystyrene (PS), polytetrafluoroethylene (PTFE), thermoplastic polyurethane (TPU), polyurethane (PU), implantable elastane polymer, polyamide, polyethylene terephthalate (PET), polyethersulfone, poly-lactic-co-glycolic acid (PLGA) or a combination thereof or natural/regenerated fibers selected from cotton, silk, linen, cellulose acetate, collagen, or a combination thereof. It is understood that the degree of perforation can be adjusted depending on the desired performance of the final materials.

In yet other aspects, any of the auxiliary layers can comprise one or more thermoplastic polyurethane (TPU), polyurethane (PU), implantable elastane polymer, or polyethylene, polypropylene, polymethylmethacrylate (PMMA), polystyrene (PS), polytetrafluoroethylene (PTFE), polyamide, polyethylene terephthalate (PET), polyethersulfone, poly-lactic-co-glycolic acid (PLGA). It is understood that any of the auxiliary layers can comprise resorbable materials, a non-resorbable materials, or any combination thereof.

It is understood that in some aspects, the first material can comprise the disclosed herein electrospun fibers and at least one perforated material and/or at least one auxiliary layer. In yet further aspects, the second material can comprise the disclosed herein electrospun fibers and at least one perforated material and/or at least one auxiliary layer, While in still further aspects, the third material can comprise the disclosed herein electrospun fibers and at least one perforated material and/or at least one auxiliary layer. It is also understood that in some aspects, some of the materials comprise all the layers, while in other aspects, some of tile materials comprise only some of the layers. It is also understood that the combination of all of the layers can be found in any or all of the first, second, and third materials.

In still further aspects, the presence of the perforated material can provide mechanical property enhancement of nanofibers while utilizing the benefits from the nanofiber structure y exposing these fibers through the porous structure.

In other aspects, any of the disclosed herein auxiliary layer s are configured to impart to at least a portion of tile first, second, and/or third material hydrophobic or hydrophilic properties, elastomeric properties, mechanical resilience, adhesive properties, tissue-iti-growth inhibition, or any combination thereof. It is understood that when for example, TPU or PU is used as an auxiliary layer at at least one portion of the inner and/or outer surface of the plurality of electrospun fibers, this portion will prohibit tissue in-growth while having increased mechanical properties.

In certain aspects, the first material and/or the second material, and/or third material can exhibit tensile strength from greater than 0 MPa to about 20 MPa, including the exemplary value of about 0.5 MPa, about 1 MPa, about 1.5 MPa, about 2 MPa, about 2.5 MPa, about 3 MPa, about 3.5 MPa, about 4 MPa, about 4.5 MPa, about 5 MPa, about 5.5 MPa, about 6 MPa, about 6.5 MPa, about 7.0 MPa, about 7.5 MPa, about 8 MPa, about 8.5 MPa, about 9.5 MPa, about 10 MPa, 10.5 MPa, about 11 MPa, about 11.5 MPa, about 12 MPa, about 12.5 MPa, about 13 MPa, about 13.5 MPa, about 14 MPa, about 14.5 MPa, about 15 MPa, about 15.5 MPa, about 16 MPa, about 16.5 MPa, about 17.0 MPa about 17.5 MPa, about 18 MPa, about 18.5 MPa, and about 19.5 MPa. Still further, the first material and/or the second material, and/or third material can exhibit tensile strength can have any value between any two foregoing values.

In certain aspects, the first material and/or the second material, and/or third material can exhibit elongation at break from greater than 0% to about 600%, including exemplary values of about 1%, about 10%, about 50%, about 100%, about 200%, about 300%, about 400%, and about 500%. Still further, the first material and/or the second material, and/or third material can exhibit elongation at break can have any value between any two foregoing values. Again it is understood that both for the tensile strength and elongation at break parameters, the skilled practitioner would choose the material having the desired properties for the specific application. For example, the first material used for the inner skirt may have properties that are different from the third material used to form the leaflet structure, and so on. It is further understood that such properties can be adjusted by changing electrospinning parameters, the average diameter of fibers, porosity, fiber composition, and the like.

In certain aspects, the first material and/or the second material, and/or third material can exhibit a water contact angle from about 0° to about 180°, including exemplary values of about 10°, about 20°, about 30°, about 40°, about 50°, about 60°, about 70°, about 80°, about 90°, about 100°, about 110°, about 120°, about 130°, about 140°, about 150°, about 160°, and about 170°. It is understood the first material and/or the second material, and/or third material can exhibit that any of the disclosed above water contact angles. It is further understood that the material having low contact angles are considered hydrophilic, and higher contact angles are considered hydrophobic. The hydrophilicity/hydrophobicity of the first material and/or the second material, and/or third material can be adjusted again by changing the density of the formed material and by chemical/physical treatment of the material to impart the desired properties to the materials. For example, hydrophobicity can be imparted to at least a portion of the fibers by exposing it to plasma. In such exemplary aspects, exposure to a 98% Helium + 2% CF₄ plasma or 99% Helium +1 % CF₄ gas plasma treatment can introduce hydrophobic groups such as fluorine groups on the fiber surfaces and to change their properties. In yet other exemplary aspects, hydrophilicity be imparted by treating the materials with 98% Helium + 2 % oxygen atmospheric pressure plasma. In such exemplary and unlimiting aspects, oxygen-free radicals can be formed. The oxygen-free radicals can attach themselves to fibers in the form of - CO-, -COOH, -COO-, -C=O, -O-COO groups and increase the hydrophilicity of a nonpolar compound. In still further exemplary aspects, CH₄ gas can cause a plasma polymerization of CH₂ polymers capped with CH₃ end groups and change the hydrophilicity of the fibers. It is understood that the plasma treatments, as shown herein, are only exemplary, and both atmospheric pressure and vacuum-based plasma treatments can be used. In still further aspects, chemical treatments can also be utilized. In such exemplary aspects, the first and/or second and/or third materials can be treated with various chemical compounds to impart desired hydrophilic or hydrophobic properties. It is understood, however, that such treatments need to be compatible with the desired applications.

In still further aspects, various portions of the same material can have different properties. For example, and without limitation, the materials can be designed to have a change in various properties along the material's dimensions. The materials can also be designed to have different properties at the surface of the material and in the bulk of the material. Such variations in the properties can be gradual or steep, depending on the final application of the material. It is understood that the properties, such as fiber density, an average fiber diameter, a pore size, and pore density, and the like, can be varied by adjusting various process parameters of the electrospinning.

In still further aspects, by adjusting the composition of the plurality of fibers according to the aspects disclosed herein, the material can be designed to have various portions of bioresorbability or biodegradability or just degradability as desired.

In yet further aspects, the frame itself can also be plasma treated to improve the adsorption of the electrospun fibers disposed thereon.

Still further and as disclosed above, the first material and/or the second material, and/or third material can be at least partially biodegradable. While in other aspects, the first material and/or the second material, and/or third material are at least partially bioresorbable. While still in further aspects, and depending on the fibers' compositions, the first material and/or the second material, and/or third material can be at least partially degradable. Also disclosed herein are aspects where the first material and/or the second material, and/or third material are a scaffold material.

Also disclosed herein is an article comprising a material comprising a plurality of fibers, wherein at least one fiber of the plurality of fibers comprises electrospun silk, wherein the article has a collapsed configuration and an expanded configuration, and wherein the article is a part of an implantable device. In certain aspects, the article can be a paravalvular leak sealing article.

Paravalvular leak (PVL) is a complication associated with the implantation of a prosthetic heart valve. PVL refers to blood flowing through a channel between the structure of the implanted valve and cardiac tissue as a result of a lack of appropriate sealing. The majority of PVL are crescent, oval, or roundish-shaped, and their track can be parallel, perpendicular, or serpiginous. Transcatheter Heart Valve (THV) procedures generally use either a substantially inelastic woven cloth or a stretchable knitted cloth for PVL sealing.

When comparing the woven material with the knitted material for PVL sealing, the substantially inelastic woven material has the advantage of providing better dimensional stability that helps in procedures dealing with joining the valve components together using sutures and laser cutting of components. Further, the pore sizes and pore densities in a woven material can be engineered to balance sealing and tissue in-growth functions. On the other hand, knitted material provides better stretchability than woven cloth construction. Stretchability helps in reducing stress on a tissue to which the medical device comprising the fibrous material is attached.

With the next generation of THV frame designs that have changing frame dimensions, one of the requirements is to have the PVL seal material and/or the frame inner material to adapt to the changing frame dimensions. Thus, there is a need for a material having controlled stretchability and a lower profile to provide improved compliance by reducing potential stresses at locations where the cloth is secured to a bodily lumen. The present disclosure is describing the aspects of utilizing electrospun silk fibers addressing the issues disclosed above. As described above, the porosity, stretchability, the physical strength of the fibers can be controlled during the single manufacturing process by varying electrospinning parameters.

The paravalvular leak sealing article, as disclosed herein, can comprise any of the disclosed above inner skirts comprising a first material comprising a plurality of fibers comprising an electrospun silk. In such aspects, the inner skirt is configured to be positioned on at least a portion of an inner surface of an annular frame of the implantable prosthetic device. The paravalvular leak sealing article, as disclosed herein, can also comprise any of the disclosed herein outer skirts comprising a second material comprising a plurality of fibers comprising an electrospun silk. In such exemplary aspects, tile outer skirt is configured to be positioned on at least a portion of an outer surface of an annular frame of the implantable prosthetic device.

The article can also comprise any of the disclosed herein leaflet structures comprising a third material comprising a plurality of fibers comprising an electrospun silk. In such aspects, the leaflet structure is configured to be positioned within at least a portion of an annular frame of the implantable prosthetic device. In still further aspects, the material can comprise any of the disclosed herein first materials, or the second materials, or the third materials, or any combination thereof.

### METHODS

The present disclosure also provides for methods of forming an implantable prosthetic valve. According to the invention, the method comprises: a) providing an annular frame having an inner surface and an outer surface wherein the frame has an inflow end and an outflow end and a central longitudinal axis extending from the inflow end to the outflow end; b) forming an inner skirt comprising a first material having a first surface and an opposite second surface and comprising a plurality of fibers, wherein at least one fiber of tile plurality of fibers comprises electrospun silk; c) forming an outer skirt comprising a second material having a first surface and an opposite second surface and comprising a plurality of fibers, wherein at least one fiber of the plurality of fibers comprises electrospun silk; d) attaching the inner skirt to at least a portion of the inner surface of the annular frame and attaching the outer skirt to at least a portion of the outer surface of the annular frame, wherein the implantable prosthetic valve is radially collapsible to a collapsed configuration and radially expandable to an expanded configuration.

In certain aspects, the step of forming the inner skirt and the step of attaching the inner skirt occurs simultaneously. While in other aspects, the step of forming tile inner skirt occurs prior to the step of attaching In yet further aspects, the step of forming the outer skirt and the step of attaching the outer skirt occurs simultaneously. While in still further aspects, the step of forming the outer skirt occurs prior to the step of attaching. In certain aspects, the step of forming the inner skirt can occur before or after the step of forming the outer skirt. Similarly, the methods described herein can further comprise a step of positioning a leaflet structure comprising a third material having a first surface and an opposite second surface and comprising a plurality of fibers, wherein at least one fiber of the plurality of fibers comprises electrospun silk within at least a portion of the annular frame, While it is also understood that the step of positioning the leaflet structure can occur before or after the step of forming tile inner skirt and/or outer skirt.

The aspects disclosed herein described electrospinning methods to form the first and/or the second and/or the third materials.

In the aspects disclosed herein, the step of attaching the at least a portion of the first material to the at least a portion of the inner surface of the annular frame comprises directly electrospinning at least a portion of the plurality of fibers through at least one spinneret from a first solution comprising a first predetermined concentration of a silk fibroin at a predetermined extrusion rate. While in other aspects, the step of attaching the at least a portion of the second material to the at least a portion of the outer surface of the annular frame comprises directly electrospinning at least a portion of the plurality of fibers through at least one spinneret from a second solution comprising a second predetermined concentration of a silk fibroin at a predetermined extrusion rate.

Also disclosed are aspects where the first and/or the second materials are not formed directly on the annular frame but formed separately and then shaped to the desired dimensions and attached to the frame with the fasteners.

For example, the step of forming the first material can comprise electrospinning at least a portion of the plurality of fibers through at least one spinneret from a first solution comprising a first predetermined concentration of a silk fibroin at a predetermined extrusion rate on a first predetermined mandrel. In such exemplary aspects, the step of attaching then comprises i) shaping the first material to a predetermined dimension and ii) attaching tile first material to the at least a portion of the inner surface of the annular frame.

Also disclosed are aspects where the step of forming the second material comprises electrospinning at least a portion of tile plurality of fibers through at least one spinneret from a second solution comprising a second predetermined concentration of a silk fibroin at a predetermined extrusion rate on a second predetermined mandrel. In such exemplary aspects, the step of attaching then comprises i) shaping the second material to a predetermined dimension and ii) attaching the second material to the at least a portion of the outer surface of the annular frame.

Still further, the third material is formed by the electrospinning of the plurality of fibers on a third predetermined mandrel from a third solution comprising a third predetermined concentration of a silk fibroin at a predetermined extrusion rate. Then the third material can be laser cut to a predetermined shape.

In certain aspects, any of the predetermined concentrations of the silk fibroin in the first, second, or the third solution can be greater than 0 to less about 50% by weight, including exemplary values of about 0.1 wt%, about 0.2 wt%, about 0.3 wt%, about 0.5 wt%, about 0.6 wt%, about 0.7 wt% about 0.8 wt%, about 0.9 wt%, about 1 wt%, about 2 wt%, about 3 wt%, about 4 wt%, about 5 wt%, about 6 wt% about 7 wt% about 8 wt%, about 9 wt%, about 10 wt%, about 1 5 wt%, about 20 wt%, about 25 wt%, about 30 wt%, about 35 wt%, about 40 wt%, and about 45 wt%. It is understood that the specific concentration can be chosen based on the desired application, and in some aspects, the concentration of silk fibroin in the first, the second, and/or the third solutions can be the same or different.

In still further aspects, any of the predetermined extrusion rate can be anywhere between 0.7 µl/hour to about 10,000 ml/hour, including exemplary values of about 0.8 µl/hour, about 1 µl/hour, about 2 µl/hour, about 5 µl/hour, about 10 µl/hour, about 20 µl/hour, about 50 µl/hour, about 100 µl/hour, about 250 µl/hour, about 500 µl/hour, about 1 ml/hour, about 10 ml/hour, about 50 ml/hour, about 100 ml/hour, about 250 ml/hour, about 500 ml/hour, about 750 ml/hour, about 1 ml/hour, about 10 ml/hour, about 50 ml/hour, about 100 ml/hour, about 250 ml/hour, about 500 ml/hour, about 750 ml/hour, about 1,000 ml/hour, about 1,250 ml/hour, about 1,500 ml/hour, about 2,000 ml/hour, about 3,000 ml/hour, about 4,000 ml/hour, about 5,000 ml/hour, about 6,000 ml/hour, about 7,000 ml/hour, about 8,000 ml/hour, about 9,000 ml/hour. It is also understood that the specific predetermined extrusion rate can be dependent on a volume of the syringe, a volume of the reservoir, pumping rate, etc. It is also understood that this parameter can be chosen on the specific applications and the component.

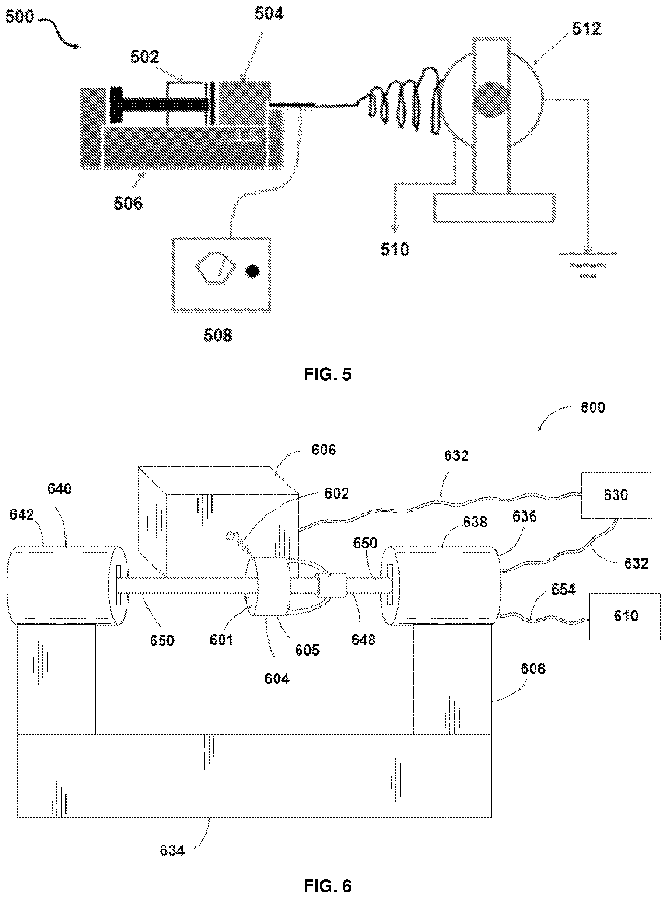

An exemplary electrospinning system **500** is shown in **FIG. 5****.** Such an exemplary system can comprise an electrospinning solution **504.** The electrospinning solution **504** can comprise silk fibroin in various concentrations. The solution is pumped with a syringe pump **506** into a syringe **502** and is extruded through the at least one spinneret ( a needle, for example) on a substrate that can be positioned on a rotational drum **512.** In this exemplary aspect, a high voltage **508** is supplied to the at least one spinneret, and the rotational drum is grounded.

**FIG. 6** shows an exemplary electrospinning system in a different aspect. This figure shows a system **600** for applying an electrospinning material **602** to a stent frame **604.** The system **600** comprises a source of electrospinning material **606,** a collector **608,** and a controller **610.** The source of electrospinning material is any suitable device, for example, a device comprising a spinneret electrically coupled to a voltage source. In some aspects, the voltage source can be electrically coupled to the at least one syringe needle. As used herein, the term "syringe pump" may include the combination of a syringe pump, syringe, and at least one syringe needle, as will be apparent by context. It is further understood, however, that needle-less spinneret systems can also be utilized. A detailed description of these aspects is enclosed below.

In these exemplary and unlimiting aspects, the source of electrospinning material can include at least one syringe pump, at least one syringe mounted on the at least one syringe pump, and at least one syringe needle fluidly coupled to the at least one syringe, where the at least one syringe needle is a spinneret. However, it is understood that this description is only exemplary and unlimiting. In certain aspects, the source of electrospinning material can comprise an assembly comprising a plurality of spinnerets. In certain aspects, the plurality of spinnerets can comprise two or more needle spinnerets. In certain exemplary aspects, these two or more needle spinnerets can be arranged concentrically to allow, for example, formation of the disclosed above bicomponent fibers. It is understood that the bicomponent fibers can comprise a side-by-side configuration, a sheath-core configuration, a tri-lobal, an islands-in-the-sea configuration, a segmented pie configuration, or any combination thereof configurations. In such aspects, the spinnerets can be configured and arrange such the desired configuration of the final fiber is obtained. In aspects where the bicomponent fibers have a sheath-core configuration, the spinnerets can be arranged concentrically, such that the inner spinneret can be connected to electrospinning solution comprising the silk fibroin, while the outer spinneret can be connected to electrospinning solution comprising any of disclosed herein polymers. It is understood that the resulting bicomponent fiber will comprise a core comprising electrospun silk fibers and a sheath comprising any of the other polymers disclosed herein. In such exemplary aspects, the bicomponent fibers are disposed by electrospinning through at least two concentric spinnerets, wherein an outer spinneret is configured to extrude a sheath fiber from a fourth solution comprising a fourth predetermined concentration of silk fibroin, and wherein an inner spinneret is configured to extrude a core fiber from a fifth solution comprising a predetermined concentration of thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof at a predetermined extrusion rate. However, it is understood that tile disclosed above procedure is only exemplary and a sheath of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and wherein a core of the bicomponent fiber can comprise one or more of silk, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluorethylene (ePTFE), polyvinylidene fluoride (PVDF), polyamides (Nylons), polyolefins such as, polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof. The predetermined extrusion can be any extrusion rate, as disclosed above,

In yet other aspects, the plurality of the needle spinneret can be arranged such that they can extrude fibers from different electrospinning solutions or the same electrospinning solution simultaneously. In such exemplary aspects, the plurality of spinneret needles can be arranged in any configuration allowing to achieve the desired result. For example, and without limitation, the plurality of needle spinnerets can be arranged in parallel or series. In still further aspects, each of the plurality of the needle spinneret can have the same diameter of the extrusion orifices, or it can be different depending on the desired result.

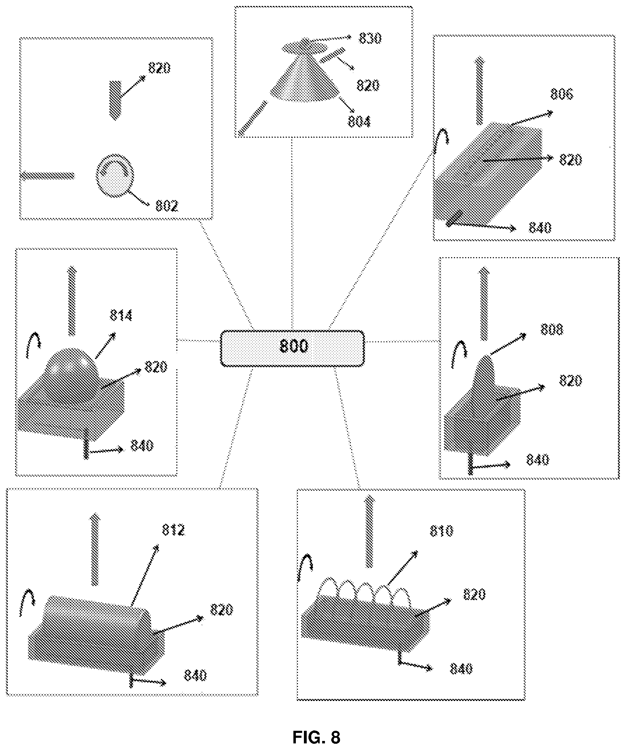

In yet further aspects, the assembly can comprise a plurality of needle-less spinnerets. In such exemplary and unlimiting aspects, various stationery and rotational needle-less spinnerets can be utilized. Any of the known in the art needle-less spinnerets can be used. Some exemplary aspects encompassing the needle-less spinneret assemblies are shown in **FIGS. 8-9****.** (https://www.hindawi.com/journls/jnm/2012/72550/)

For example, **FIG. 8** summarizes the rotating spinnerets **802, 804, 806, 808, 810, 812, 814** that are all connected with a high voltage power supply **840** and a spinning solution **820.** For cylinder **812,** ball **814,** disc **808,** coil **810,** and beaded chain **806** electrospinning, spinnerets can be immersed in the electrospinning solutions comprising any of the disclosed herein polymers. Nanofibers are electrospun upward on the desired support. In such aspects, the support can comprise any of the disclosed herein frames that can be positioned directly on rotational drums or have been handled with specially designed holders and/or spaces. The rotation of spinnerets conveys polymer solutions to the electrospinning sites, ensuring the production continuously. For roller **802** and cone **804** (which is spun using a DC motor **830)** electrospinning, the spinning solutions are fed from separated solution containers.

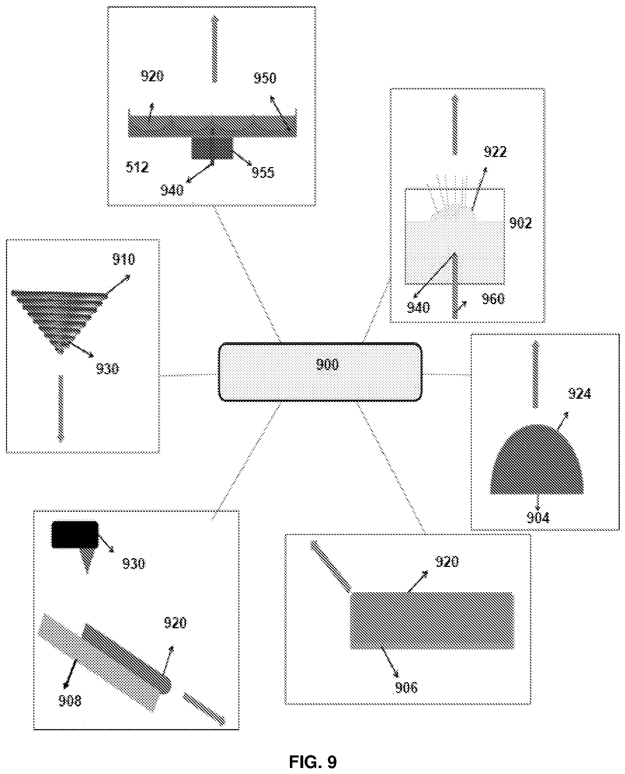

It is understood that needleless electrospinning can be dependent on the initiation of jets from an open liquid surface. When stationary spinnerets are employed, conical spikes are often created with the aid of an external force, such as magnetic force, high-pressure gas flow, and gravity. The known in the art stationary needle-less spinnerets that can be used in disclosed herein aspects are shown in **FIG. 9****.** For example, the reservoir **902** comprising the electrospinning solution can be electrically connected to a high voltage **940,** a high-pressure nitrogen gas **960** is purged into the solution to create solution bubbles **922** and initiate electrospinning. A stationary cylinder **904** spinneret having a solution layer **924** on top of it can also be utilized. Additional examples can include a bowl spinneret **906** with electrospinning solution **920;** a plate spinneret **908** can be connected gravitationally with the solution reservoir **930** to obtain an electrospinning solution **920;** a conical wire spinneret **910** having an electrospinning solution **930** flowing through the cone; or a reservoir spinneret comprising a magnet **955** connected to a high voltage **940** and having a magnetic fluid **950** and electrospinning solution **920.** It is understood, however, that such needleless spinnerets are only exemplary and unlimiting any other known in the art spinnerets can be utilized.

It is further understood that a type of the spinnerets used to form the disclosed herein materials can be chosen based on the desired application or scalability of the process. It is understood that in comparison with the needle electrospinning, the needle-less spinnerets can provide a higher output of the fibers ( for example, the cylinder spinneret can provide about 8.6 g/hr, the disc spinneret can provide about 6.2 g/hr, and the ball spinneret can provide about 3.1 g/hr).

Still further, a specific choice of the spinneret can be determined by additional parameters. In some aspects, if the plurality of fibers having a finer average diameter is desired, the disc needle-less spinneret can be used (257 ± 77 nm). Such a spinneret can provide fibers with a narrower diameter distribution compared to the ball (344 ± 105 nm) and the cylinder (357 ± 127 nm) spinnerets.

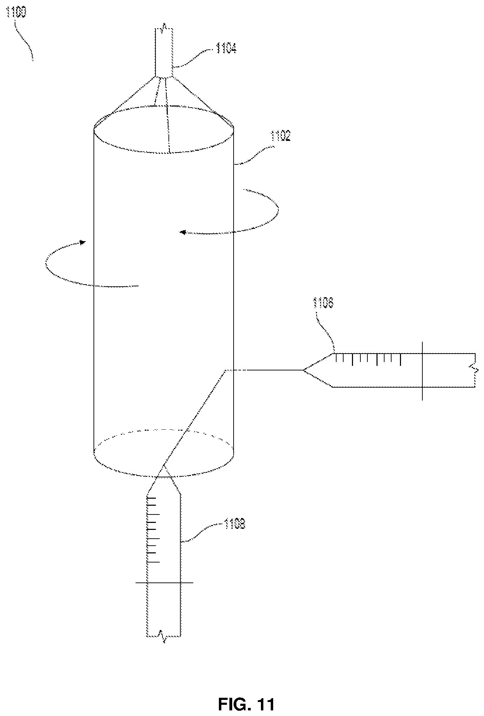

In still further aspects, the extrusion spinnerets can also be positioned within at least a portion of an inner space of the annular frame, where the inner space is defined by a circumference of the inner surface of the annular frame. In such exemplary aspects, at least one additional extrusion spinneret is also positioned outside of the annular frame. Such an exemplary aspect is shown in **FIG. 11****.** Referring to **FIG. 11****,** one can see a setup **1100** that comprises an annular frame **1102,** a holder **1104** configured to hold the annular frame **1102** at a predetermined height and configured to allow the annular frame **1102** to rotate in the desired orientation and speed. In this exemplary setup, at least one extrusion spinneret **1108** is positioned within the inner space of the annular frame a third distance from the annular frame and is configured to be moved within the inner space of the annular frame. In yet other aspects, the folder **1104** is configured to move the annular frame relatively to the spinneret **1108** to achieve the desired third distance. An additional extrusion spinneret **1106** is positioned outside of the annular frame at a fourth predetermined distance. It is understood that the third and the fourth predetermined distances can be adjustable by moving the extrusion spinnerets and/or the annular frame relative to each other.

In certain aspects, the electrospinning can occur simultaneously from the spinneret **1108** and **1106.** While in other aspects, the fibers first electrospun from the spinneret **1106** and the from the spinneret **1108.** However, it is understood that also the aspects, where the fibers are first electrospun from the spinneret **1108** and then spinneret **1106,** are also disclosed. In some aspects, the electrospinning of the fibers can be done in a cycling manner. For example, and without limitations, a cycle of the electrospinning from the spinneret **1108** is followed by electrospinning from the spinneret **1106** and then followed again by the electrospinning from the spinneret **1108,** etc. It is understood that the reverse order of using the spinnerets is also disclosed It is also understood that the duration of each cycle can be determined by specific properties of the desired fibers, material thickness, material density and the like.

In some aspects, the spinnerets **1108** and **1106** can be connected to the same pump. While in other aspects, the spinnerets **1108** and **1106** can be separately assembled. It is understood that additional spinnerets can also be added to the inner space of the annular frame and/or outside of the annular frame. It is also understood that any of the disclosed herein spinnerets can be utilized.

Referring back to **FIG. 6****.** In one aspect, the electrospinning material **602** is a solution of a silk fibroin (SF). The SF solution may be created by mixing SF, for example, at about greater than 0 to less than 100% by weight, with a suitable solvent or mixture of solvents, such as, for example, 2,2-Trifluoroacetic acid (TFA) solvent or forming acid, or a mixture of formic acid and CaCl₂, or a mixture of hexafluoroisopropanol (HFIP) and formic acid.

It is understood, however, that this is only an exemplary solvent, and any other solvents can be used. The concentration of tile silk fibroin in the solvent can be greater than 0 to less about 50% by weight, including exemplary values of about 0.1 wt%, about 0.2 wt%, about 0.3 wt%, about 0.5 wt%, about 0.6 wt%, about 0.7 wt%, about 0.8 wt% about 0.9 wt%, about 1 wt%, about 2 wt%, about 3 wt%, about 4 wt%, about 5 wt%, about 6 wt%, about 7 wt%, about 8 wt%, about 9 wt% about 10 wt%, about 15 wt% about 20 wt%, about 25 wt%, about 30 wt%, about 35 wt%, about 40 wt%, and about 45 wt%. It is understood that tile silk fibroin can be presented in any value between any foregoing values. In certain aspects, the silk fibroin can be fully dissolved in tile solvent. While in other aspects, the silk fibroin can form a saturated solution. It is also understood that with an increase in polymer concentration, the average diameter of the fibers will also increase.

Still further and as disclosed herein, any one of the disclosed herein polymers can also be added to the solution of the silk fibroin in any desired concentration. For example, thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), polyvinylidene fluoride (PVDF), polyamides (Nylons), polyolefins such as polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof can be added to the solution comprising the silk filbroin. Any of the disclosed polymers can also be dissolved in any known in the art solvents. For example, the solvents used herein can comprise 2,2-Trifluoroacetic acid (TFA), dichloromethane (DCM), chloroform, methanol, formic acid, acetic acid, or chlorophenol, or any combinations thereof. It is also understood, and as described herein, in certain aspects, one or more separate electrospinning solutions can be utilized. In such aspects, any of tile disclosed herein polymers can be present in these separate electrospinning solutions. The use of the separate electrospinning solutions having various combinations of the polymers and their concentration can allow a more precise control of tile desired properties of tile materials used to form the disclosed herein implantable devices. Additionally, one or more drugs and/or biologically active ingredients can be added to any of the described herein solutions.

In one exemplary aspect, the stent **604** can be any frame disclosed herein or known in the art. The stent **604** may be an expandable stainless-steel stent, or it can be a polymeric stent or it can be a nitinol stent. As disclosed, the material is not limited and can also include other materials such as cobalt-chrome alloys, for example.

The exemplary syringe pump **606** serves as the source of the electrospinning material **602** to be applied to frame **604.** As disclosed in detail above, some aspects can include a plurality of syringe pumps. In general, electrospinning uses an electrical charge to draw very fine (typically on the micro- or nanometer scale) fibers from a liquid, such as a polymer solution or a polymer melt. In one electrospinning method, the polymer is discharged through a charged orifice toward a target, wherein the orifice and the target have opposing electrical charges. A voltage source is provided that creates a first charge at the charged orifice and an opposing charge at the target. The polymer is electrostatically charged by contact with the charged orifice. The electrostatically charged polymer is then collected at the target. Electrospinning of other than silk fibroin materials such as PET and PTFE are described in U.S. Patent Application Publication No. 2017/0325976 A1 and U.S. Patent Application Publication No. 2010/0193999 A1, which contents of both are incorporated herein by reference in their full entirety. Also, various aspects of the syringe pump **606,** syringe needle, collector **608,** controller 610, or mandrel **648** are disclosed in U.S. Patent Application Publication No. 2017/0325976 A1, which content is incorporated herein by reference in its full entirety.

The electrospinning material **602** is electrostatically drawn from the spinneret tip (not shown) by placing or applying a high voltage or potential difference between the spinneret tip and the collector **608** using a high-voltage power supply **630** connected by wires **632** to the spinneret and the collector. In one aspect, the high-voltage power supply **630** is an about 5 kV to 50 kV, including exemplary values of about 10 kV, about 15 kV, about 20 kV, about 25 kV, about 30 kV, about 35 kV, about 40 kV, and about 45 kV, direct-current power supply. In a particular aspect, the high-voltage power supply **630** is configured to apply any voltage within the described value to achieve the desired results.

It is understood that in certain aspects, the diameter of the fibers, their porosity, and mechanical strength can also be controlled by an amount of voltage applied to the system and by a specific polarity applied to tile collector and the spinneret. It is understood that the higher the applied voltage, the average diameter of the obtained fiber will be smaller. Also, in some exemplary aspects, when the collector has a negative polarity, and the spinneret has a positive polarity, the finer fibers having a substantially uniform diameter (smaller standard deviation) can be obtained.

Referring back to **FIG. 6****.** In this exemplary aspect, the collector **608** can comprise a base **634** configured to hold a rotary tool **636** at a first end **638** and a rotary holder **640** at a second end **642.** An exemplary mandrel **648** can be placed in the collector **608** by placing a first end **650** of the mandrel into the rotary holders and tools.

In certain aspects, the exemplary electrospinning systems, as shown in **FIGS. 5** and **6** can be used to form the inner and outer skirts. The frames can be placed on the rotational drum directly or using mandrels or any known in the art holders. Such various holders and spacers are disclosed in U.S. Patent Publication Number 2017/0325976 and U.S. Patent Application No. 62/882,352, which are incorporated herein by reference.

**FIG. 7A** shows an exemplary electrospinning system **700** that can be used to produce the leaflet structure. In such an exemplary and unlimiting aspect, the solution reservoir **702** comprising any of the disclosed above polymers can be utilized. A high voltage **709** can be applied to the needle spinneret **704,** and fiber can be electrospun on a mandrel **706** comprising having the desired shape, for example, the shape of the leaflet structure. In certain aspects, the leaflet structure can also be formed by forming the third material by electrospinning any of the disclosed herein a plurality of fibers to obtain a sheet like fibrous material (**FIG. 7B**). The desired leaflet structure can then be laser or ultrasonically cut out. It is understood that using a laser or ultrasonic cutter is only exemplary, and any other methods can be used. It is also understood that in certain aspects, the inner and/or outer skirts can also be formed from the sheet like fibrous material. In such aspects, the cut out inner and/or outer skirts can then be attached to the frame with a fastener. In such exemplary aspects, the fastener can comprise a glue or sutures, or any other known in the art and suitable fasteners.

Also, it is understood that the properties of the electrospun materials can be varied by varying various electrospinning parameters. For example, in certain aspects during the electrospinning of the at least a portion of the plurality of fibers to form the first material and/or the second material, the at least a portion of the inner surface or the outer surface, depending on the application, of the annular frame is positioned at a first predetermined distance or at a second predetermined distance, respectively from the at least one extrusion spinneret. Similarly, in other aspects, during the electrospinning of the at least a portion of the plurality of fibers to form the third material, the at least a portion of a third predetermined mandrel is positioned at a third predetermined distance from the at least one extrusion spinneret. The first, second, and/or predetermined distance can be the same or different, depending on the desired properties and can range from about 0.1 cm to about 200 cm, including exemplary values of 0.5 cm, about 1 cm, about 5 cm, about 10 cm, about 20 cm, about 50 cm, about 100 cm, about 125 cm, about 150 cm, and about 175 cm. It is understood that at shorter distances, larger fibers can be obtained.

In certain aspects, and as disclosed herein, at least a portion of the annular frame can be positioned on a rotational drum configured to rotate at a predetermined speed. In such aspects, the predetermined speed can be from greater than 0 rpm to about 1,200 rpm, including exemplary values of about 5 rpm, about 10 rpm, about 20 rpm, about 50 rpm, about 100 rpm, about 200 rpm, about 300 rpm, about 400 rpm, about 500 rpm, about 600 rpm, about 700 rpm, about 800 rpm, about 900 rpm, about 1,000 rpm, and about 1,110 rpm. In still further aspects, the mandrel used to form the leaflet system can be stationary or rotational. If the mandrel is rotational, the predetermined speed of the mandrel can also be from greater than 0 rpm to about 1,200 rpm, including exemplary values of about 5 rpm, about 10 rpm, about 20 rpm, about 50 rpm, about 100 rpm, about 200 rpm, about 300 rpm, about 400 rpm, about 500 rpm, about 600 rpm, about 700 rpm, about 800 rpm, about 900 rpm, about 1,000 rpm, and about 1,110 rpm. It is further understood that the mechanical and scaffold properties of the formed materials can also be controlled by varying the rotating speed of the drum. For example, when the drum/mandrel is rotated slowly, the plurality of fibers can have a random orientation. While in other aspects, when the drum/mandrel is rotated faster, a more aligned orientation of the plurality of fibers can be obtained.

In still further aspects, it is understood that a plurality of various parameters can be used at once to control the desired mechanical and scaffold properties of the materials disclosed herein. For example, in some aspects, a first plurality of fibers can be formed at higher drum rotations combined with the higher voltages and farther distances, then a second plurality of fibers can be formed by slowing the drum rotations and/or decreasing the voltage and/or shortening the distance between the drum collector and the extrusion spinneret. It is understood that such manipulations of the process conditions can be used to obtain the desired average diameter of the fibers and the porosity of the formed materials.

It is also understood that the materials formed by the disclosed methods can be scaffold materials. The growth of the cells on the disclosed herein materials can also be controlled by controlling the average diameter of the fibers and the porosity of the material. Similarly, the skilled practitioner can tune the biodegradation and bioresolabitlity rate of the formed materials by precisely control and optimization of the average diameter of the fibers and the porosity.

In still further aspects, to increase the mechanical strength of the fibers, semi-liquid electrospinning processes can be utilized. In such exemplary aspects, a first plurality of layers can be electrospun when the collector is positioned at a distance close enough that a solvent present in the electrospinning solution does not evaporate before the electrospun fibers are formed on the collector. In such an exemplary aspect, the formed fibers are "semi-liquid." Still further, after forming the first plurality of layers, the collector can be moved to a distance far enough to allow a solvent present in the electrospinning solution to evaporate before the electrospun fibers are formed on the collector and to form "dry fibers." Such a sequence of forming different layers can be repeated as needed. Yet, in other aspects, a similar effect can be obtained by varying the extrusion rate. For example, and without limitation, the first plurality of layers can be deposited with a high extrusion rate, followed by forming the second plurality of layers deposited with a low extrusion rate, etc. Without wishing to be bound by any theory, it is hypothesized that during "semi-liquid"-"dry" electrospinning, a residual solvent penetrates various layers and improves the interconnectivity of the layers and, therefore, the overall strength of the material.

In still further aspects, to improve the mechanical strengths of the first, the second, and/or the third material, these materials can be chemically or physically treated. In some aspects, and as disclosed above, plasma treatment of the material can be applied. In such exemplary aspects, the plasma treatment can activate free radicals on the substrate, which then crosslinks and forms bonds with the depositing layer or amongst a polymeric chain of depositing nanofibers layers to increase the strength and change hydrophilicity/hydrophobicity of the material as disclosed above.

Yet, in further aspects, heat treatment can be used. While in yet other aspects, a freezing and thawing process can be utilized. In still further aspects, controllable welding can be used to improve the mechanical properties of the materials.

Still further and disclosed herein, prior to the step attaching the at least a portion of the first and/or the second material to the at least a portion of the annular frame, an adhesive material is applied to the at least a portion of the annular frame. While in other aspects, prior to forming the inner skirt and/or the outer skirt, at least a portion of the annular frame can be plasma treated.

As disclosed herein, the undulations of the plurality of the fibers can also be formed.

In still further aspects and as disclosed herein, the first, second, and/or third materials can be formed on separate and predetermined mandrels and then shaped to the desired dimensions.

In some of the aspects, any or all of the first, second, and/or third materials can further comprise at least one perforated material. In such aspects, the electrospun plurality of fibers can be disposed of the at least one perforated material.

It is understood that the at least one perforated material can be disposed on any surface of the electrospun plurality of fibers. In some aspects, for example, when the inner skirt is formed, the at least one perforated material can be disposed on the surface facing the annular frame. While in other aspects, it can be disposed on the surface facing the inner portion of the frame. Similarly, in the case of forming the outer skirt the perforated material can be disposed on the surface facing the annular frame or the surface facing the natural anatomy of the subject.

In yet other aspects, the third material can also comprise at least one perforated material that can be disposed on any or both of the surfaces of the third material.

It is understood that any combinations of the various configurations can be formed. For example, the first material can comprise at least one perforated material, while the second and the third materials are not. Yet, in other examples, the only second material can comprise at least one perforated material. While in still other examples, both the first and the second materials can comprise at least one perforated material. In a still further example, all three materials can comprise the at least one perforated material, and so on. In still further aspects, two or more perforated materials can be utilized. In some aspects, the plurality of fibers can be sandwiched between the perforated materials. In such aspects, the two perforated materials and the plurality of fibers can be coupled to each other. In still further aspects, two or more layers of the perforated material can be used on each or any surface of the disclosed herein materials. Any of the disclosed herein perforated materials can be utilized.

In some aspects, the plurality of the fibers of the first, second, and/or third materials can be directly electrospun on the at least one perforated material. Yet, in other aspects, the plurality of the fibers of the first, second, and/or third materials can be attached to the at least one perforated material by any known in the art means. In some aspects, the attachment can be done with a fastener, for example, an adhesive or a suture. In yet other aspects, the plurality of fibers can be heat pressed to the perforated materials. In some aspects, the attachment can be done at any portion of the perforated material. For example, in some aspects, the attachment can be done throughout all surfaces of the perforated material. While in other aspects, the attachment can be done only on one or more edges of the perforated material or anywhere on the surface of the perforated material in any desired pattern. It is understood that the placing of attachment and the amount of the surface that is physically attached to the plurality of fibers can vary depending on the desired applications.

In still further aspects and as disclosed above, the first, the second, and/or third materials can also comprise at least one auxiliary layer. Any of the disclosed above auxiliary layers can be utilized. Similarly, the auxiliary layer can be disposed at any surface of any of the disclosed herein materials. In some aspects, the auxiliary layer can be coated, can be sprayed, solution deposited, or otherwise applied by any known in the art methods. In some aspects, both the perforated material and auxiliary layer are present. In such aspects, the attachment between all the layers can be done throughout the whole material, one or more edges, or in any pattern as desired.

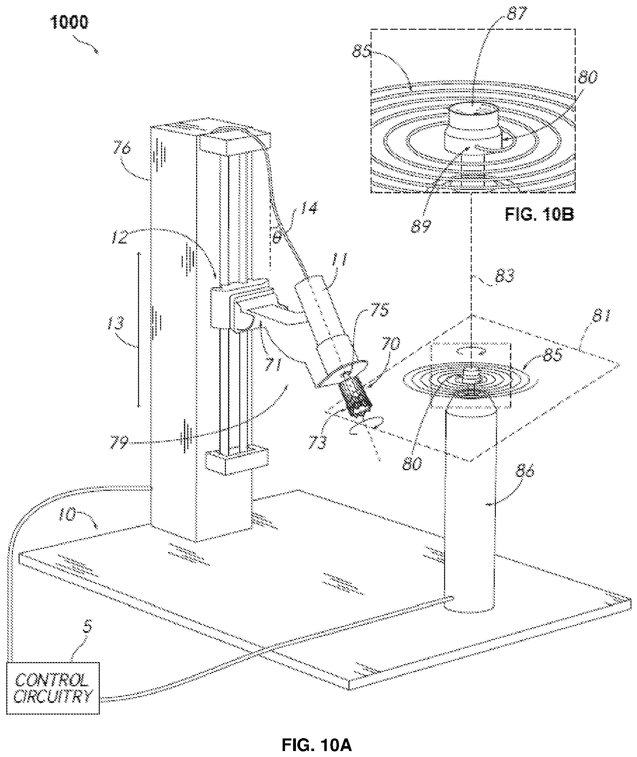

An alternative aspect of forming the disclosed implantable devices and materials is shown in **FIG. 10A-10B****.** In such implementations, the fibrous material can be applied to a medical implant device using a rotary jet spinning process and not electrospinning. For example, with respect to certain prosthetic heart valve implant devices, the fibrous material can be applied to a metal stent structure, wherein the applied fibrous material may serve to reduce friction between the stent and certain anatomy (e.g., vessel/orifice) at the implantation site, to secure the implant device at the implantation site, to fill gaps through which fluid may pass, and/or to provide a surface for tissue in-growth. Rotary jet spinning application of fibrous material represents another example of a method of applying a fabric or fibrous material (e.g., polymeric fibrous material) to surfaces of a stent or other medical device implant component in a way that can reduce labor time and production costs. By way of illustration, rotary-jet-spun material may be applied to a medical device implant (e.g., metal stent) while the implant and a supporting holder are rotated by a rotary tool. Over time, the rotary jet spinning process can produce a layer of polymeric threads or fibers covering the outside of the target surface. Rotary jet spinning generally does not require the use of any electric field, unlike electrospinning. Rotary jet spinning, as described in greater detail below, can involve conversion of a material (e.g., polymer) dissolved in a solvent into a continuous fibrous strand/fiber by centrifugal ejection of the material/solvent at high speed, such that the ejected strand/fiber at least partially coats or is otherwise applied to a target surface. For example, the target surface may comprise a surface of a medical device component (e.g., stent/frame), which may be rotated as well to cover a varying surface area. Certain methods, devices, and systems relating to rotary jet spinning concepts that may be applicable to aspects of the present disclosure are disclosed in U.S. Patent No. 9,41 0,267.

Rotary jet spinning systems and processes can involve imparting rotational motion to a reservoir holding any of the disclosed above polymer solutions, the rotational motion causing the polymer to be ejected from one or more orifices in the reservoir. Such processes can further involve collecting the formed fibers on a holder having a desired shape to form micron-, submicron- or nanometer-dimensioned polymeric fibers as a covering for component(s) of a medical implant device component. **FIG. 10A** shows a system **1000** for applying a rotary jet spinning material **85** to a stent or other medical implant device component **73** coupled to a holder component **70** that is associated with a rotating mandrel **75.** The system 1000 can comprise a rotary motor (e.g., pneumatic motor) **86,** which may be configured to drive the rotation of a reservoir **80.** Reservoir **80** is shown in close-up in **FIG. 10B****.** In some aspects, the polymer solution is extruded through a small orifice **89.** The extrusion of the solution can produce a plane **81** of fibers **85** into which the rotating holder **70** is translated into and out of during the collection process in the desired translation sequence.

The rotation of the mandrel **75** and holder **70** can be driven by a motor **11.** Furthermore, the mandrel **75** and holder **70** may be mounted on a linear motor **12** configured to effect vertical translation of the mandrel **75** and holder **70.** The motor **12** can be considered a fiber plane translation motor and may comprise, for example, a high uniaxial precision linear drive that is configured to translate the collector assembly **79** along an axis **13** parallel to the rotation axis **83** of the rotating reservoir **80,** which corresponds to vertical translation with respect to the illustrated orientation of **FIG. 10A****.** Axis **83** may be referred to as the deposition rotation axis. In some aspects, one or more additional linear drives can be employed to translate the rotating mandrel **75** and holder **70** along one or more axes perpendicular to the rotation axis **83** of the rotating reservoir(s) (*e.g*., movement toward and away from the deposition rotation axis **83**). In some aspects, a multi-axial drive or a robotic arm could be employed to provide increased flexibility in translation and/or changing an angular alignment of the holder **70.**

The mandrel **75** and holder **70** can represent components of the collection assembly **79,** at least part of which can be inserted into the path/plane **81** of the polymeric fibers **85.** Axis **14,** about which the mandrel/holder **70** is rotated, may be referred to as the collection rotation axis or mandrel/holder rotation axis. When the holder **70** is in the path/plane **81** of the polymeric fibers **85** ejected from the rotating reservoir **80,** the polymeric fibers **85** can become wrapped around the holder **70** via rotation of the holder **70** about the collection rotation axis **14** as the holder **70** is translated along the axis **13.**

In some aspects, methods of depositing fibrous material on a medical implant device component involve feeding any of the disclosed herein polymers into the rotating reservoir **80** and generating rotational motion at a speed, and for a time, sufficient to form a micron-, submicron-, or nanometer-dimensioned polymeric fiber, and collecting the formed fibers on a medical implant device to form the micron-, submicron-, or nanometer-dimensioned polymeric fiber covering in the desired shape/configuration. In some aspects, fibrous strands are produced by subjecting the polymer solution to a sufficient amount of pressure/stress for a time sufficient to form a fibrous covering on one or more components of a medical implant device in the desired shape and/or configuration. For example, sufficient pressure/stress to produce fibrous strands from the polymer solution can be about 3,000 Pascals or more.

In some aspects, the system **1000** is at least partially automated by control circuitry **5** configured to control one or more of the rotation rate of the reservoir **80,** the rotation rate of the holder **70,** and the linear and/or multi-dimensional translation of the holder **70** along the axis **13** parallel to the rotation axis **83** of the rotating reservoir and/or one or more other axes, through the generation and/or transmission of electrical signals to one or more components of the system **1000.**

Control over the rate of translation of the holder **70** along the axis **13** and/or the orientation of the collection axis **14** relative to the reservoir rotation axis **83** can provide at least partial control over the orientation of fibers deposited on the collection holder **70.** For example, fibers may be collected on the holder **70** substantially parallel to the reservoir rotation axis **83** and with slow translation along the collection rotation axis **14.** In some implementations, the rotation of the collection device (*e.g*., holder **70**) may be opposite the rotation of the reservoir **80** (*e.g*., counter-clockwise and clockwise, respectively), or the rotation of the collection device **70** may be the same as the rotation of the reservoir **80** (e.g., both counter-clockwise). In some implementations, by slowly moving the collection device (*e.g*., holder **70**) along axis **13** through a path of the polymeric fibers **85** while rotating the collection device/assembly **70,** completely aligned coverage of the holder and/or medical device component held thereby.

As shown in **FIG. 10A****,** the collection rotation axis **14** can be oriented at an angle *θ* with respect to the deposition rotation axis. Such a configuration may result in fiber collection on the collection assembly **70** with crossed polymeric fibers. By increasing the speed of translation and/or rotating the holder **70** at a nonzero angle *θ* with respect to the deposition rotation axis, crossed weaves can be produced. The collection assembly, as disclosed herein, can be moved manually or mechanically.

In some aspects, the system **1000** includes a platform **10** for supporting the deposit of fibrous material, wherein the deposition assembly (**80, 86**) and the collection assembly (**70, 71, 73, 76, 11**) are disposed vertically above the platform **10** and/or spaced from the platform **10** along the vertical axis **13.** Sufficient rotational speeds and times for operating the rotating structure **80** to form a fiber may be dependent on the concentration of the material/solution and the desired features of the formed fiber. Exemplary speeds of rotation of the rotating structure may range from about 100 rpm to about 500,000 rpm, although rotational speeds are not limited to this exemplary range. Furthermore, the rotating structure **80** may be rotated to impact the liquid material for a time sufficient to form a desired fiber, such as, for example, an amount of time between about 1-100 minutes, or other intermediate times or ranges are also intended to be part of this disclosure. The force or energy imparted by the rotating structure **80** advantageously overcomes the surface tension of the solution and decouples a portion of the liquid material at a meniscus thereof, and flings the portion away from the contact with the rotating structure and from a platform (not shown) on which the liquid is maintained, thereby forming fiber(s). The fiber(s) can be collected on the collection device **70.** In some aspects, the direction in which the liquid material is flung may be substantially the same as the tangential direction of motion of the rotating structure of the reservoir **80** that contacts the liquid material. In some aspects, the rotating structure may impart a force to the liquid material in a substantially parallel direction to the top surface of the liquid material.

Any suitable size or geometrically-shaped reservoir **80** or collector **70** may be used for fabricating/collecting polymeric fibers. For example, reservoir **80** may be tubular, conical, semilunar, bicuspid, round, rectangular, or oval. The holder **70** may be round, oval, rectangular, or a half-heart shape. The holder **70** may also be shaped in the form of any living organ, such as a heart, kidney, liver lobe(s), bladder, uterus, intestine, skeletal muscle, or lung shape, or portion thereof. The holder **70** may further be shaped as any hollow cavity, organ or tissue, such as a circular muscle structure, e.g., a valve, sphincter, or iris.

The collection device **70** may be a holder configured in the desired shape and positioned in the path of the polymer ejected from the one or more orifices or in the path of the fibers flung from the rotating structure **80.** In some aspects, the collection device **70** may be disposed at a distance of about 2 inches (about 5 cm) to about 12 inches (about 30 cm) from the reservoir **80** from which the polymer is ejected. Certain exemplary distances may include, but are not limited to 2, 3, 4, 5, 6, 7, 8, 9, 10, 11, 12 inches (5, 7.6, 10.2, 12.7, 15.2, 17.8, 20.3, 22.9, 25.4, 27.9, 30 cm), and all intermediate numbers. This distance may be selected and/or configured to avoid formation of fibrous beads (which may occur if the collection device **70** is too close to the reservoir **80**) and to achieve sufficient fibrous mass (which may not occur if the collection device is too far from the reservoir). In some implementations, the formation of fibrous beads is implemented intentionally to provide desired fiber characteristics. Still, other exemplary aspects of forming various implantable devices utilizing rotary jet spinning systems can be found in U.S. Patent Application No. 62/882,352.

Although several aspects of the disclosure have been disclosed in the foregoing specification, it is understood by those skilled in the art that many modifications and other aspects of the disclosure will come to mind to which the disclosure pertains, having the benefit of the teaching presented in the foregoing description and associated drawings.

## Claims

1. An implantable prosthetic valve (10) comprising:
an annular frame (1200) having an inner surface and an outer surface, wherein the frame has an inflow end (16) and an outflow end (18), and a central longitudinal axis (24) extending from the inflow end to the outflow end;
a leaflet structure (140) positioned within the frame (1200);
an inner skirt positioned along the inner surface of the frame (1200);
at least one outer skirt (30) positioned around the outer surface of the frame (1200);
wherein at least a portion of one of the leaflet structure (140), the inner skirt, or the at least one outer skirt (30) comprises a material comprising a plurality of fibers (1304), wherein at least one fiber of the plurality of fibers comprises electrospun silk; and
wherein the implantable prosthetic valve (10) is radially collapsible to a collapsed configuration and radially expandable to an expanded configuration.

2. The implantable prosthetic valve of claim 1, wherein:
a) at least a portion of the inner skirt comprises the material comprising the plurality of fibers (1304), and wherein the material present in the at least a portion of the inner skirt is a first material, wherein the first material has a first surface facing the annular frame (1200) and an opposite second surface, and/or
b) at least a portion of the outer skirt (30) comprises the material comprising the plurality of fibers (1304), and wherein the material present in the at least a portion of the outer skirt is a second material, wherein the second material has a first surface facing the annular frame (1200) and an opposite second surface, and/or
c) at least a portion of the leaflet structure (140) comprises the material comprising the plurality of fibers (1304), and wherein the material present in the at least a portion of the leaflet structure is a third material, wherein the third material has a first surface facing the annular frame (1200) and an opposite second surface.

3. The implantable prosthetic valve of claim 1 or claim 2, wherein each fiber of the plurality of fibers (1304) has a first extending direction and a plurality of undulations,
optionally wherein the first extending direction comprises a circumferential direction, a radial direction, or a combination thereof; and
optionally wherein the plurality of undulations are:
a) present in the collapsed configuration, and/or
b) configured to straighten when the implantable prosthetic valve (10) is in the expanded configuration.

4. The implantable prosthetic valve of any one of claims 1-3, wherein:
a) at least a portion of the inner skirt is attached to the annular frame by direct electrospinning of the plurality of fibers on at least a portion of the inner surface of the annular frame, and/or
b) at least a portion of the outer skirt is attached to at least a portion of the annular frame by direct electrospinning of the plurality of fibers on at least a portion of the outer surface of the annular frame.

5. The implantable prosthetic valve of any one of claims 1-4, wherein the plurality of fibers (1304):
a) further comprise a resorbable material, a non-resorbable material, or any combination thereof; and/or
b) further comprises at least one fiber comprising thermoplastic polyurethane (TPU), polyurethane (PU), implantable elastane polymer, polyester (PET), ultra-high molecular weight polyethylene (UHMWPE), polytetrafluorethylene (PTFE), expanded polytetrafluoroethylene (ePTFE), Polyvinylidene fluoride (PVDF), Polyamides (Nylons), polypropylene, polyetheretherketone (PEEK), polyglycolic acid (PGA), poly(ester urethane)urea, polylactic acid (PLA), polycaprolactone (PCL), poly(lactic-co-glycolic acid) (PLGA) or any combination thereof; and/or
c) comprises a bicomponent fiber.

6. The implantable prosthetic valve of any one of claims 2-5, wherein:
a) at least a portion of the inner skirt further comprises a first perforated material having a first surface facing the annular frame (1200) and an opposite second surface and wherein the first material is disposed on the first surface and/or the second surface of the first perforated material, and/or
b) at least a portion of the outer skirt (30) further comprises a second perforated material having a first surface facing the annular frame (1200) and an opposite second surface and wherein the second material is disposed on the first surface and/or the second surface of the second perforated material, and/or
c) at least a portion of the leaflet structure (140) comprises a third perforated material having a first surface facing the annular frame (1200) and an opposite second surface and wherein the third material is disposed on the first surface and/or the second surface of the third perforated material;
optionally wherein the first, second, and/or third perforated material comprises a porous fabric or membrane, wherein the porous fabric or membrane comprises one or more biocompatible polymers that are resorbable, non-resorbable or a combination thereof.

7. The implantable prosthetic valve of any one of claims 2-6, wherein:
a) at least a portion of the first surface of the first material comprises a first auxiliary layer and/or wherein at least a portion of the second surface of the first material comprises a first auxiliary layer, optionally wherein the first auxiliary layer present on the second surface of the first material is the same or different as the first auxiliary layer present on the first surface of the first material, and/or
b) at least a portion of the first surface of the second material comprises a second auxiliary layer and/or wherein at least a portion of the second surface of the second material comprises a second auxiliary layer, optionally wherein the second auxiliary layer present on the second surface of the second material is the same or different as the second auxiliary layer present on the first surface of the second material; and/or
c) at least a portion of the first surface of the third material comprises a third auxiliary layer and/or wherein at least a portion of the second surface of the third material comprises a third auxiliary layer, optionally wherein the third auxiliary layer present on the second surface of the third material is the same or different as the third auxiliary layer present on the first surface of the third material; and
optionally wherein the first, second, and/or third auxiliary layer comprises one or more thermoplastic polyurethane (TPU), polyurethane (PU); implantable elastane polymer, or polyethylene, polypropylene, polymethylmethacrylate (PMMA), polystyrene (PS), polytetrafluoroethylene (PTFE),
polyamide, polyethylene terephthalate (PET), polyethersulfone, poly-lactic-co-glycolic acid (PLGA).

8. A method of forming an implantable prosthetic valve (10) comprising:
a) providing an annular frame (1200) having an inner surface and an outer surface wherein the frame has an inflow end (16) and an outflow end (18) and a central longitudinal axis (24) extending from the inflow end to the outflow end;
b) forming an inner skirt comprising a first material having a first surface and an opposite second surface and comprising a plurality of fibers (1304), wherein at least one fiber of the plurality of fibers comprises electrospun silk;
c) forming an outer skirt (30) comprising a second material having a first surface and an opposite second surface and comprising a plurality of fibers (1304), wherein at least one fiber of the plurality of fibers comprises electrospun silk;
d) attaching the inner skirt to at least a portion of the inner surface of the annular frame (1200) and attaching the outer skirt to at least a portion of the outer surface of the annular frame, and
wherein the implantable prosthetic valve (10) is radially collapsible to a collapsed configuration and radially expandable to an expanded configuration.

9. The method of claim 8, wherein:
a) the step of forming the inner skirt and the step of attaching the inner skirt occurs simultaneously; or
b) the step of forming the inner skirt occurs prior to the step of attaching.

10. The method of claim 8 or claim 9, wherein:
a) the step of forming the outer skirt and the step of attaching the outer skirt occurs simultaneously; or
b) the step of forming the outer skirt occurs prior to the step of attaching.

11. The method of any one of claims 8 - 10, further comprising positioning a leaflet structure (140) comprising a third material having a first surface and an opposite second surface and comprising a plurality of fibers (1304), wherein at least one fiber of the plurality of fibers comprises electrospun silk within at least a portion of the annular frame (1200);
optionally wherein:
a) the third material is formed by the electrospinning of the plurality of fibers on a third predetermined mandrel from a third solution comprising a third predetermined concentration of a silk fibroin at a predetermined extrusion rate; and/or
b) at least a portion of the leaflet structure is disposed on a third perforated material having a first surface and an opposite second surface and wherein the third material is disposed on the first surface and/or the second surface of the third perforated material, optionally comprising disposing the third material between two layers of the third perforated material; and wherein at the two layers of the third perforated material are at least partially coupled to each other; and/or
c) the method comprises disposing a third auxiliary layer at at least a portion of the first surface of the third material and/or at at least a portion of the second surface of the third material.

12. The method of claim 9a) or or claim 10 or 11 insofar as they depend on claim 9a), wherein the step of simultaneously forming and attaching the inner skirt to the at least a portion of the inner surface of the annular frame (1200) comprises forming the first material by directly electrospinning at least a portion of the plurality of fibers (1304) through at least one spinneret from a first solution comprising a first predetermined concentration of a silk fibroin at a predetermined extrusion rate at the at least a portion of the inner surface of the annular frame.

13. The method of claim 9b) or claim 10 or 11 insofar as they depend on claim 9b), wherein:
a) the step of forming the first material comprises electrospinning at least a portion of the plurality of fibers through at least one spinneret from a first solution comprising a first predetermined concentration of a silk fibroin at a predetermined extrusion rate on a first predetermined mandrel, optionally wherein the step of attaching comprises i) shaping the first material to a predetermined dimension and ii) attaching the first material to the at least a portion of the inner surface of the annular frame; and/or
b) at least a portion of the formed first material is disposed on a first perforated material having a first surface and an opposite second surface prior to the step of attaching, and wherein the first material is disposed on the first surface and/or the second surface of the first perforated material; optionally comprising disposing the first material between two layers of the first perforated material, and wherein the two layers of the first perforated material are at least partially coupled to each other; and/or
c) the method comprises disposing a first auxiliary layer at at least a portion of the first surface of the first material and/or at at least a portion of the second surface of the first material.

14. The method of claim 10a) or claim 11 insofar as it depends on claim 10a), wherein the step of simultaneously forming and attaching the outer skirt (30) to the at least a portion of the outer surface of the annular frame (1200) comprises forming the second material by directly electrospinning at least a portion of the plurality of fibers (1304) through at least one spinneret from a second solution comprising a second predetermined concentration of a silk fibroin at a predetermined extrusion rate the at least a portion of the outer surface of the annular frame.

15. The method of claim 10b) or claim 11 insofar as it depends on claim 10b), wherein:
a) the step of forming the second material comprises electrospinning at least a portion of the plurality of fibers through at least one spinneret from a second solution comprising a second predetermined concentration of a silk fibroin at a predetermined extrusion rate on a second predetermined mandrel, optionally wherein the step of attaching comprises i) shaping the second material to a predetermined dimension and ii) attaching the second material to the at least a portion of the outer surface of the annular frame; and/or
b) at least a portion of the formed second material is disposed on a second perforated material having a first surface and an opposite second surface, the prior to the step of attaching, and wherein the second material is disposed on the first surface and/or the second surface of the second perforated material; optionally comprising the second material disposed between two layers of the second perforated material and wherein at the two layers of the second perforated material are at least partially coupled to each other; and/or
c) the method comprises disposing a second auxiliary layer at at least a portion of the first surface of the second material and/or at at least a portion of the second surface of the second material.

16. The method of any one of claims 12-15, wherein:
a) the electrospinning is performed by cycling; and/or
b) during the electrospinning of the at least a portion of the plurality of fibers (1304) to form the first material and/or the second material and/or the third material, the at least a portion of the inner surface of the annular frame (1200) and/or the at least a portion of the outer surface of the annular frame and/or the at least a portion of the first, second and/or third predetermined mandrels are positioned at a distance from the at least one extrusion spinneret such that the distance is varied during the electrospinning to form one or more plurality of layers within at least a portion of the first material and/or the second material and/or the third material.

17. The method of any one of claims 12-16, wherein:
a) the at least one spinneret comprises a needle; or
b) the at least one spinneret is a part of an assembly comprising a plurality of spinnerets, optionally wherein the assembly comprises a plurality of needle-less spinnerets.

## Patentansprüche

1. Implantierbare künstliche Herzklappe (10), umfassend:
einen ringförmigen Rahmen (1200) mit einer Innenfläche und einer Außenfläche, wobei der Rahmen ein Einströmungsende (16) und ein Ausströmungsende (18) sowie eine zentrale Längsachse (24), die sich vom Einströmungsende zum Ausströmungsende erstreckt, aufweist,
eine Segelstruktur (140), die innerhalb des Rahmens (1200) positioniert ist,
eine innere Schürze, die entlang der Innenfläche des Rahmens (1200) positioniert ist,
mindestens eine äußere Schürze (30), die um die Außenfläche des Rahmens (1200) herum positioniert ist, wobei mindestens ein Teil der Segelstruktur (140), der inneren Schürze oder der mindestens einen äußeren Schürze (30) ein Material umfasst, das eine Vielzahl von Fasern (1304) umfasst, wobei mindestens eine Faser aus der Vielzahl von Fasern elektrogesponnene Seide umfasst, und wobei die implantierbare künstliche Herzklappe (10) radial zu einer zusammengeklappten Auslegung zusammenklappbar und radial zu einer expandierten Auslegung expandierbar ist.

2. Implantierbare künstliche Herzklappe nach Anspruch 1, wobei:
a) mindestens ein Teil der inneren Schürze das Material umfasst, das die Vielzahl von Fasern (1304) umfasst, und wobei das Material, das in dem mindestens einen Teil der inneren Schürze vorhanden ist, ein erstes Material ist, wobei das erste Material eine erste Oberfläche, die dem ringförmigen Rahmen (1200) zugewandt ist, und eine gegenüberliegende zweite Oberfläche aufweist, und/oder
b) mindestens ein Teil der äußeren Schürze (30) das Material umfasst, das die Vielzahl von Fasern (1304) umfasst, und wobei das Material, das in dem mindestens einen Teil der äußeren Schürze vorhanden ist, ein zweites Material ist, wobei das zweite Material eine erste Oberfläche, die dem ringförmigen Rahmen (1200) zugewandt ist, und eine gegenüberliegende zweite Oberfläche aufweist, und/oder
c) mindestens ein Teil der Segelstruktur (140) das Material umfasst, das die Vielzahl von Fasern (1304) umfasst, und wobei das Material, das in dem mindestens einen Teil der Segelstruktur vorhanden ist, ein drittes Material ist, wobei das dritte Material eine erste Oberfläche, die dem ringförmigen Rahmen (1200) zugewandt ist, und eine gegenüberliegende zweite Oberfläche aufweist.

3. Implantierbare künstliche Herzklappe nach Anspruch 1 oder Anspruch 2, wobei jede Faser aus der Vielzahl von Fasern (1304) eine erste Erstreckungsrichtung und eine Vielzahl von Wellen aufweist,
wobei optional die erste Erstreckungsrichtung eine Umfangsrichtung, eine radiale Richtung oder eine Kombination davon umfasst und
wobei optional die Vielzahl von Wellen:
a) in der zusammengeklappten Auslegung vorhanden ist und/oder
b) dazu ausgelegt ist, sich geradezurichten, wenn sich die implantierbare künstliche Herzklappe (10) in der expandierten Auslegung befindet.

4. Implantierbare künstliche Herzklappe nach einem der Ansprüche 1 bis 3, wobei:
a) mindestens ein Teil der inneren Schürze an dem ringförmigen Rahmen durch direktes Elektrospinnen der Vielzahl von Fasern auf mindestens einem Teil der Innenfläche des ringförmigen Rahmens angebracht ist und/oder
b) mindestens ein Teil der äußeren Schürze an mindestens einem Teil des ringförmigen Rahmens durch direktes Elektrospinnen der Vielzahl von Fasern auf mindestens einem Teil der Außenfläche des ringförmigen Rahmens angebracht ist.

5. Implantierbare künstliche Herzklappe nach einem der Ansprüche 1 bis 4, wobei die Vielzahl von Fasern (1304):
a) ferner ein resorbierbares Material, ein nicht resorbierbares Material oder eine beliebige Kombination davon umfasst und/oder
b) ferner mindestens eine Faser umfasst, die thermoplastisches Polyurethan (TPU), Polyurethan (PU), implantierbares Elastanpolymer, Polyester (PET), ultrahochmolekulares Polyethylen (UHMWPE), Polytetrafluorethylen (PTFE), expandiertes Polytetrafluorethylen (ePTFE), Polyvinylidenfluorid (PVDF), Polyamide (Nylons), Polypropylen, Polyetheretherketon (PEEK), Polyglykolsäure (PGA), Poly(esterurethan)Harnstoff, Polymilchsäure (PLA), Polycaprolacton (PCL), Poly(lactic-co-glycolid) (PLGA) oder eine beliebige Kombination davon umfasst und/oder
c) eine Zweikomponentenfaser umfasst.

6. Implantierbare künstliche Herzklappe nach einem der Ansprüche 2 bis 5, wobei:
a) mindestens ein Teil der inneren Schürze ferner ein erstes perforiertes Material mit einer ersten Oberfläche, die dem ringförmigen Rahmen (1200) zugewandt ist, und einer gegenüberliegenden zweiten Oberfläche umfasst, und wobei das erste Material auf der ersten Oberfläche und/oder der zweiten Oberfläche des ersten perforierten Materials angeordnet ist, und/oder
b) mindestens ein Teil der äußeren Schürze (30) ferner ein zweites perforiertes Material mit einer ersten Oberfläche, die dem ringförmigen Rahmen (1200) zugewandt ist, und einer gegenüberliegenden zweiten Oberfläche umfasst, und wobei das zweite Material auf der ersten Oberfläche und/oder der zweiten Oberfläche des zweiten perforierten Materials angeordnet ist, und/oder
c) mindestens ein Teil der Segelstruktur (140) ein drittes perforiertes Material mit einer ersten Oberfläche, die dem ringförmigen Rahmen (1200) zugewandt ist, und einer gegenüberliegenden zweiten Oberfläche umfasst, und wobei das dritte Material auf der ersten Oberfläche und/oder der zweiten Oberfläche des dritten perforierten Materials angeordnet ist,
wobei optional das erste, zweite und/oder dritte perforierte Material ein poröses Gewebe oder eine poröse Membran umfasst, wobei das poröse Gewebe oder die poröse Membran ein oder mehrere biokompatible Polymere umfasst, die resorbierbar, nicht resorbierbar oder eine Kombination davon sind.

7. Implantierbare künstliche Herzklappe nach einem der Ansprüche 2 bis 6, wobei:
a) mindestens ein Teil der ersten Oberfläche des ersten Materials eine erste Hilfsschicht umfasst und/oder wobei mindestens ein Teil der zweiten Oberfläche des ersten Materials eine erste Hilfsschicht umfasst, wobei optional die erste Hilfsschicht, die auf der zweiten Oberfläche des ersten Materials vorhanden ist, mit der ersten Hilfsschicht, die auf der ersten Oberfläche des ersten Materials vorhanden ist, identisch oder von ihr verschieden ist und/oder
b) mindestens ein Teil der ersten Oberfläche des zweiten Materials eine zweite Hilfsschicht umfasst und/oder wobei mindestens ein Teil der zweiten Oberfläche des zweiten Materials eine zweite Hilfsschicht umfasst, wobei optional die zweite Hilfsschicht, die auf der zweiten Oberfläche des zweiten Materials vorhanden ist, mit der zweiten Hilfsschicht, die auf der ersten Oberfläche des zweiten Materials vorhanden ist, identisch oder von ihr verschieden ist, und/oder
c) mindestens ein Teil der ersten Oberfläche des dritten Materials eine dritte Hilfsschicht umfasst und/oder wobei mindestens ein Teil der zweiten Oberfläche des dritten Materials eine dritte Hilfsschicht umfasst, wobei optional die dritte Hilfsschicht, die auf der zweiten Oberfläche des dritten Materials vorhanden ist, mit der dritten Hilfsschicht, die auf der ersten Oberfläche des dritten Materials vorhanden ist, identisch oder von ihr verschieden ist, und
wobei optional die erste, zweite und/oder dritte Hilfsschicht ein oder mehrere thermoplastische Polyurethane (TPU), Polyurethane (PU); implantierbare Elastanpolymere oder Polyethylene, Polypropylene, Polymethylmethacrylate (PMMA), Polystyrole (PS), Polytetrafluorethylene (PTFE), Polyamide, Polyethylenterephthalate (PET), Polyethersulfone, Poly(lactic-co-glycolide) (PLGA) umfasst.

8. Verfahren zum Bilden einer implantierbaren künstlichen Herzklappe (10), umfassend:
a) Bereitstellen eines ringförmigen Rahmens (1200) mit einer Innenfläche und einer Außenfläche, wobei der Rahmen ein Einströmungsende (16) und ein Ausströmungsende (18) sowie eine zentrale Längsachse (24) aufweist, die sich von dem Einströmungsende zu dem Ausströmungsende erstreckt,
b) Bilden einer inneren Schürze, die ein erstes Material mit einer ersten Oberfläche und einer gegenüberliegenden zweiten Oberfläche umfasst und eine Vielzahl von Fasern (1304) umfasst, wobei mindestens eine Faser aus der Vielzahl von Fasern elektrogesponnene Seide umfasst,
c) Bilden einer äußeren Schürze (30), die ein zweites Material mit einer ersten Oberfläche und einer gegenüberliegenden zweiten Oberfläche umfasst und eine Vielzahl von Fasern (1304) umfasst, wobei mindestens eine Faser aus der Vielzahl von Fasern elektrogesponnene Seide umfasst,
d) Anbringen der inneren Schürze an mindestens einem Teil der Innenfläche des ringförmigen Rahmens (1200) und Anbringen der äußeren Schürze an mindestens einem Teil der Außenfläche des ringförmigen Rahmens und
wobei die implantierbare künstliche Herzklappe (10) radial zu einer zusammengeklappten Auslegung zusammenklappbar und radial zu einer expandierten Auslegung expandierbar ist.

9. Verfahren nach Anspruch 8, wobei:
a) der Schritt des Bildens der inneren Schürze und der Schritt des Anbringens der inneren Schürze gleichzeitig erfolgen oder
b) der Schritt des Bildens der inneren Schürze vor dem Schritt des Anbringens erfolgt.

10. Verfahren nach Anspruch 8 oder 9, wobei:
a) der Schritt des Bildens der äußeren Schürze und der Schritt des Anbringens der äußeren Schürze gleichzeitig erfolgen oder
b) der Schritt des Bildens der äußeren Schürze vor dem Schritt des Anbringens erfolgt.

11. Verfahren nach einem der Ansprüche 8 bis 10, ferner umfassend das Positionieren einer Segelstruktur (140), die ein drittes Material mit einer ersten Oberfläche und einer gegenüberliegenden zweiten Oberfläche umfasst und eine Vielzahl von Fasern (1304) umfasst, wobei mindestens eine Faser aus der Vielzahl von Fasern elektrogesponnene Seide innerhalb mindestens eines Teils des ringförmigen Rahmens (1200) umfasst,
wobei optional:
a) das dritte Material durch Elektrospinnen der Vielzahl von Fasern auf einem dritten vorgegebenen Dorn aus einer dritten Lösung, die eine dritte vorgegebene Konzentration eines Seidenfibroins umfasst, mit einer vorgegebenen Extrusionsrate gebildet wird und/oder
b) mindestens ein Teil der Segelstruktur auf einem dritten perforierten Material mit einer ersten Oberfläche und einer gegenüberliegenden zweiten Oberfläche angeordnet ist und wobei das dritte Material auf der ersten Oberfläche und/oder der zweiten Oberfläche des dritten perforierten Materials angeordnet ist, optional umfassend das Anordnen des dritten Materials zwischen zwei Schichten des dritten perforierten Materials, und wobei die zwei Schichten des dritten perforierten Materials mindestens teilweise miteinander gekoppelt sind, und/oder
c) das Verfahren das Anordnen einer dritten Hilfsschicht an mindestens einem Teil der ersten Oberfläche des dritten Materials und/oder an mindestens einem Teil der zweiten Oberfläche des dritten Materials umfasst.

12. Verfahren nach Anspruch 9a) oder Anspruch 10 oder 11, soweit sie von Anspruch 9a) abhängig sind, wobei der Schritt des gleichzeitigen Bildens und Anbringens der inneren Schürze an dem mindestens einen Teil der Innenfläche des ringförmigen Rahmens (1200) das Bilden des ersten Materials durch direktes Elektrospinnen mindestens eines Teils der Vielzahl von Fasern (1304) durch mindestens eine Spinndüse aus einer ersten Lösung, die eine erste vorgegebene Konzentration eines Seidenfibroins umfasst, mit einer vorgegebenen Extrusionsrate an dem mindestens einen Teil der Innenfläche des ringförmigen Rahmens umfasst.

13. Verfahren nach Anspruch 9b) oder Anspruch 10 oder 11, soweit sie von Anspruch 9b) abhängig sind, wobei:
a) der Schritt des Bildens des ersten Materials das Elektrospinnen mindestens eines Teils der Vielzahl von Fasern durch mindestens eine Spinndüse aus einer ersten Lösung, die eine erste vorgegebene Konzentration eines Seidenfibroins umfasst, mit einer vorgegebenen Extrusionsrate auf einem ersten vorgegebenen Dorn umfasst, wobei optional der Schritt des Anbringens i) das Formen des ersten Materials auf eine vorgegebene Abmessung und ii) das Anbringen des ersten Materials an dem mindestens einen Teil der Innenfläche des ringförmigen Rahmens umfasst und/oder
b) mindestens ein Teil des gebildeten ersten Materials auf einem ersten perforierten Material mit einer ersten Oberfläche und einer gegenüberliegenden zweiten Oberfläche vor dem Schritt des Anbringens angeordnet wird und wobei das erste Material auf der ersten Oberfläche und/oder der zweiten Oberfläche des ersten perforierten Materials angeordnet wird, optional umfassend das Anordnen des ersten Materials zwischen zwei Schichten des ersten perforierten Materials, und wobei die zwei Schichten des ersten perforierten Materials mindestens teilweise miteinander gekoppelt sind und/oder
c) das Verfahren das Anordnen einer ersten Hilfsschicht an mindestens einem Teil der ersten Oberfläche des ersten Materials und/oder an mindestens einem Teil der zweiten Oberfläche des ersten Materials umfasst.

14. Verfahren nach Anspruch 10a) oder Anspruch 11, soweit er von Anspruch 10a) abhängig ist, wobei der Schritt des gleichzeitigen Bildens und Anbringens der äußeren Schürze (30) an dem mindestens einen Teil der Außenfläche des ringförmigen Rahmens (1200) das Bilden des zweiten Materials durch direktes Elektrospinnen mindestens eines Teils der Vielzahl von Fasern (1304) durch mindestens eine Spinndüse aus einer zweiten Lösung, die eine zweite vorgegebene Konzentration eines Seidenfibroins umfasst, mit einer vorgegebenen Extrusionsrate des mindestens einen Teils der Außenfläche des ringförmigen Rahmens umfasst.

15. Verfahren nach Anspruch 10b) oder Anspruch 11, soweit er von Anspruch 10b) abhängig ist, wobei:
a) der Schritt des Bildens des zweiten Materials das Elektrospinnen mindestens eines Teils der Vielzahl von Fasern durch mindestens eine Spinndüse aus einer zweiten Lösung, die eine zweite vorgegebene Konzentration eines Seidenfibroins umfasst, mit einer vorgegebenen Extrusionsrate auf einem zweiten vorgegebenen Dorn umfasst, wobei optional der Schritt des Anbringens i) das Formen des zweiten Materials auf eine vorgegebene Abmessung und ii) das Anbringen des zweiten Materials an dem mindestens einen Teil der Außenfläche des ringförmigen Rahmens umfasst und/oder
b) mindestens ein Teil des gebildeten zweiten Materials vor dem Schritt des Anbringens auf einem zweiten perforierten Material mit einer ersten Oberfläche und einer gegenüberliegenden zweiten Oberfläche angeordnet wird, und wobei das zweite Material auf der ersten Oberfläche und/oder der zweiten Oberfläche des zweiten perforierten Materials angeordnet wird, optional umfassend das zweite Material, das zwischen zwei Schichten des zweiten perforierten Materials angeordnet ist, und wobei die zwei Schichten des zweiten perforierten Materials mindestens teilweise miteinander gekoppelt sind, und/oder
c) das Verfahren das Anordnen einer zweiten Hilfsschicht an mindestens einem Teil der ersten Oberfläche des zweiten Materials und/oder an mindestens einem Teil der zweiten Oberfläche des zweiten Materials umfasst.

16. Verfahren nach einem der Ansprüche 12 bis 15, wobei:
a) das Elektrospinnen durch Takten erfolgt und/oder
b) während des Elektrospinnens des mindestens einen Teils der Vielzahl von Fasern (1304), um das erste Material und/oder das zweite Material und/oder das dritte Material zu bilden, des mindestens einen Teils der Innenfläche des ringförmigen Rahmens (1200) und/oder des mindestens einen Teils der Außenfläche des ringförmigen Rahmens und/oder des mindestens einen Teils des ersten, zweiten und/oder dritten vorgegebenen Dorns in einem Abstand von der mindestens einen Extrusionsdüse auf eine solche Weise positioniert werden, dass der Abstand während des Elektrospinnens variiert wird, um eine oder mehrere Vielzahlen von Schichten innerhalb mindestens eines Teils des ersten Materials und/oder des zweiten Materials und/oder des dritten Materials zu bilden.

17. Verfahren nach einem der Ansprüche 12 bis 16, wobei:
a) die mindestens eine Spinndüse eine Nadel umfasst oder
b) die mindestens eine Spinndüse ein Teil einer Anordnung ist, die eine Vielzahl von Spinndüsen umfasst, wobei die Anordnung optional eine Vielzahl von nadellosen Spinndüsen umfasst.

## Revendications

1. Valvule prothétique implantable (10) comprenant :
un cadre annulaire (1200) ayant une surface interne et une surface externe, le cadre ayant une extrémité d'entrée (16) et une extrémité de sortie (18), et un axe longitudinal central (24) s'étendant de l'extrémité d'entrée à l'extrémité de sortie ;
une structure de feuillet (140) positionnée à l'intérieur du cadre (1200) ;
ne jupe interne positionnée le long de la surface interne du cadre (1200) ;
au moins une jupe externe (30) positionnée autour de la surface externe du cadre (1200) ;
au moins une partie d'une de la structure de feuillet (140), de la jupe interne ou de la jupe externe (30) comprenant un matériau comprenant une pluralité de fibres (1304), au moins une fibre de la pluralité de fibres comprenant de la soie électrofilée ; et
la valvule prothétique implantable (10) pouvant être repliée radialement dans une configuration repliée et radialement dilatée dans une configuration déployée.

2. Valvule prothétique implantable selon la revendication 1,
a) au moins une partie de la jupe interne comprenant le matériau comprenant la pluralité de fibres (1304), et le matériau présent dans au moins une partie de la jupe interne étant un premier matériau, le premier matériau ayant une première surface faisant face au cadre annulaire (1200) et une seconde surface opposée, et/ou
b) au moins une partie de la jupe externe (30) comprenant le matériau comprenant la pluralité de fibres (1304), et le matériau présent dans l'au moins une partie de la jupe externe étant un deuxième matériau, le deuxième matériau ayant une première surface faisant face au cadre annulaire (1200) et une seconde surface opposée, et/ou
c) au moins une partie de la structure de feuillet (140) comprenant le matériau comprenant la pluralité de fibres (1304), et le matériau présent dans l'au moins une partie de la structure de feuillet étant un troisième matériau, le troisième matériau ayant une première surface faisant face au cadre annulaire (1200) et une seconde surface opposée.

3. Valvule prothétique implantable selon la revendication 1 ou la revendication 2, chaque fibre de la pluralité de fibres (1304) ayant une première direction d'extension et une pluralité d'ondulations, éventuellement, la première direction d'extension comprenant une direction circonférentielle, une direction radiale ou une combinaison de celles-ci ; et éventuellement, la pluralité d'ondulations étant :
a) présente dans la configuration repliée, et/ou
b) configurée pour se redresser lorsque la valvule prothétique implantable (10) est dans la configuration déployée.

4. Valvule prothétique implantable selon l'une quelconque des revendications 1 à 3,
a) au moins une partie de la jupe interne étant fixée au cadre annulaire par électrofilage direct de la pluralité de fibres sur au moins une partie de la surface interne du cadre annulaire, et/ou
b) au moins une partie de la jupe externe étant fixée à au moins une partie du cadre annulaire par électrofilage direct de la pluralité de fibres sur au moins une partie de la surface externe du cadre annulaire.

5. Valvule prothétique implantable selon l'une quelconque des revendications 1 à 4, la pluralité de fibres (1304) :
a) comprenant en outre un matériau résorbable, un matériau non résorbable, ou toute combinaison de ceux-ci ; et/ou
b) comprenant en outre au moins une fibre comprenant du polyuréthane thermoplastique (TPU), du polyuréthane (PU), un polymère d'élasthanne implantable, du polyester (PET), du polyéthylène de poids moléculaire ultra élevé (UHMWPE), du polytétrafluoréthylène (PTFE), du polytétrafluoréthylène expansé (ePTFE), du fluorure de polyvinylidène (PVDF), des polyamides (Nylons), du polypropylène, du polyétheréthercétone (PEEK), de l'acide polyglycolique (PGA), du poly(ester uréthane)urée, de l'acide polylactique (PLA), du polycaprolactone (PCL), du poly(acide lactique-co-glycolique) (PLGA) ou toute combinaison de ceux-ci ; et/ou
c) comprenant une fibre bicomposant.

6. Valvule prothétique implantable selon l'une quelconque des revendications 2 à 5,
a) au moins une partie de la jupe interne comprenant en outre un premier matériau perforé ayant une première surface faisant face au cadre annulaire (1200) et une seconde surface opposée, et le premier matériau étant disposé sur la première surface et/ou la seconde surface du premier matériau perforé, et/ou
b) au moins une partie de la jupe externe (30) comprenant en outre un deuxième matériau perforé ayant une première surface faisant face au cadre annulaire (1200) et une seconde surface opposée, et le deuxième matériau étant disposé sur la première surface et/ou la seconde surface du deuxième matériau perforé, et/ou
c) au moins une partie de la structure de feuillet (140) comprenant un troisième matériau perforé ayant une première surface faisant face au cadre annulaire (1200) et une seconde surface opposée, et le troisième matériau étant disposé sur la première surface et/ou la seconde surface du troisième matériau perforé ; éventuellement, le premier, le deuxième et/ou le troisième matériau perforé comprenant un tissu ou une membrane poreux, le tissu ou la membrane poreux comprenant un ou plusieurs polymères biocompatibles qui sont résorbables, non résorbables ou une combinaison de ceux-ci.

7. Valvule prothétique implantable selon l'une quelconque des revendications 2 à 6,
a) au moins une partie de la première surface du premier matériau comprenant une première couche auxiliaire et/ou au moins une partie de la seconde surface du premier matériau comprenant une première couche auxiliaire, éventuellement la première couche auxiliaire présente sur la seconde surface du premier matériau étant identique ou différente de la première couche auxiliaire présente sur la première surface du premier matériau, et/ou
b) au moins une partie de la première surface du deuxième matériau comprenant une deuxième couche auxiliaire et/ou au moins une partie de la seconde surface du deuxième matériau comprenant une deuxième couche auxiliaire, éventuellement la deuxième couche auxiliaire présente sur la seconde surface du deuxième matériau étant identique ou différente de la deuxième couche auxiliaire présente sur la première surface du deuxième matériau ; et/ou
c) au moins une partie de la première surface du troisième matériau comprenant une troisième couche auxiliaire et/ou au moins une partie de la seconde surface du troisième matériau comprenant une troisième couche auxiliaire, éventuellement la troisième couche auxiliaire présente sur la seconde surface du troisième matériau étant identique ou différente de la troisième couche auxiliaire présente sur la première surface du troisième matériau ; et
éventuellement, la première, la deuxième et/ou la troisième couche auxiliaire comprenant un ou plusieurs parmi du polyuréthane thermoplastique (TPU), du polyuréthane (PU), un polymère d'élasthanne implantable, ou du polyéthylène, du polypropylène, du polyméthacrylate de méthyle (PMMA), du polystyrène (PS), du polytétrafluoroéthylène (PTFE),
du polyamide, du polyéthylène téréphtalate (PET), polyéthersulfone, de l'acide poly-lactique-co-glycolique (PLGA).

8. Procédé de formation d'une valvule prothétique implantable (10) comprenant :
a) la fourniture d'un cadre annulaire (1200) ayant une surface interne et une surface externe, le cadre ayant une extrémité d'entrée (16) et une extrémité de sortie (18) et un axe longitudinal central (24) s'étendant de l'extrémité d'entrée à l'extrémité de sortie ;
b) la formation d'une jupe interne comprenant un premier matériau ayant une première surface et une seconde surface opposée et comprenant une pluralité de fibres (1304), au moins une fibre de la pluralité de fibres comprenant de la soie électrofilée ;
c) la formation d'une jupe externe (30) comprenant un deuxième matériau ayant une première surface et une seconde surface opposée et comprenant une pluralité de fibres (1304), au moins une fibre de la pluralité de fibres comprenant de la soie électrofilée ;
d) la fixation de la jupe interne à au moins une partie de la surface interne du cadre annulaire (1200) et la fixation de la jupe externe à au moins une partie de la surface externe du cadre annulaire, et
la valvule prothétique implantable (10) pouvant être repliée radialement dans une configuration repliée et radialement dilatée dans une configuration déployée.

9. Procédé selon la revendication 8,
a) l'étape de formation de la jupe interne et l'étape de fixation de la jupe interne ayant lieu simultanément ; ou
b) l'étape de formation de la jupe interne ayant lieu avant l'étape de fixation.

10. Procédé selon la revendication 8 ou selon la revendication 9,
a) l'étape de formation de la jupe externe et l'étape de fixation de la jupe externe ayant lieu simultanément ; ou
b) l'étape de formation de la jupe externe ayant lieu avant l'étape de fixation.

11. Procédé selon l'une quelconque des revendications 8 à 10, comprenant en outre le positionnement d'une structure de feuillet (140) comprenant un troisième matériau ayant une première surface et une seconde surface opposée et comprenant une pluralité de fibres (1304), au moins une fibre de la pluralité de fibres comprenant de la soie électrofilée à l'intérieur d'au moins une partie du cadre annulaire (1200) ; éventuellement :
a) le troisième matériau étant formé par électrofilage de la pluralité de fibres sur un troisième mandrin prédéterminé à partir d'une troisième solution comprenant une troisième concentration prédéterminée d'une fibroïne de soie à une vitesse d'extrusion prédéterminée ; et/ou
b) au moins une partie de la structure de feuillet étant disposée sur un troisième matériau perforé ayant une première surface et une seconde surface opposée et le troisième matériau étant disposé sur la première surface et/ou la seconde surface du troisième matériau perforé, comprenant éventuellement la disposition du troisième matériau entre deux couches du troisième matériau perforé ; et les deux couches du troisième matériau perforé étant au moins partiellement couplées l'une à l'autre ; et/ou
c) le procédé comprenant la disposition d'une troisième couche auxiliaire sur au moins une partie de la première surface du troisième matériau et/ou sur au moins une partie de la seconde surface du troisième matériau.

12. Procédé selon la revendication 9a) ou la revendication 10 ou 11 dans la mesure où elles dépendent de la revendication 9a), l'étape de formation et de fixation simultanées de la jupe interne à au moins une partie de la surface interne du cadre annulaire (1200) comprenant la formation du premier matériau par électrofilage direct d'au moins une partie de la pluralité de fibres (1304) à travers au moins une filière à partir d'une première solution comprenant une première concentration prédéterminée d'une fibroïne de soie à une vitesse d'extrusion prédéterminée au niveau de l'au moins une partie de la surface interne du cadre annulaire.

13. Procédé selon la revendication 9b) ou la revendication 10 ou 11 dans la mesure où elles dépendent de la revendication 9b),
a) l'étape de formation du premier matériau comprenant l'électrofilage d'au moins une partie de la pluralité de fibres à travers au moins une filière à partir d'une première solution comprenant une première concentration prédéterminée d'une fibroïne de soie à une vitesse d'extrusion prédéterminée sur un premier mandrin prédéterminé, l'étape de fixation comprenant éventuellement i) la mise en forme du premier matériau à une dimension prédéterminée et ii) la fixation du premier matériau à l'au moins une partie de la surface interne du cadre annulaire ; et/ou
b) au moins une partie du premier matériau formé étant disposée sur un premier matériau perforé ayant une première surface et une seconde surface opposée avant l'étape de fixation, et le premier matériau étant disposé sur la première surface et/ou la seconde surface du premier matériau perforé ; éventuellement la disposition du premier matériau entre deux couches du premier matériau perforé, et les deux couches du premier matériau perforé étant au moins partiellement couplées l'une à l'autre ; et/ou
c) le procédé comprenant la disposition d'une première couche auxiliaire sur au moins une partie de la première surface du premier matériau et/ou sur au moins une partie de la seconde surface du premier matériau.

14. Procédé selon la revendication 10a) ou la revendication 11 dans la mesure où elle dépend de la revendication 10a), l'étape de formation et de fixation simultanées de la jupe externe (30) à au moins une partie de la surface externe du cadre annulaire (1200) comprenant la formation du deuxième matériau par électrofilage direct d'au moins une partie de la pluralité de fibres (1304) à travers au moins une filière à partir d'une deuxième solution comprenant une deuxième concentration prédéterminée d'une fibroïne de soie à une vitesse d'extrusion prédéterminée sur au moins une partie de la surface externe du cadre annulaire.

15. Procédé selon la revendication 10b) ou la revendication 11 dans la mesure où elle dépend de la revendication 10b)
a) l'étape de formation du deuxième matériau comprenant l'électrofilage d'au moins une partie de la pluralité de fibres à travers au moins une filière à partir d'une deuxième solution comprenant une deuxième concentration prédéterminée d'une fibroïne de soie à une vitesse d'extrusion prédéterminée sur un deuxième mandrin prédéterminé, l'étape de fixation comprenant éventuellement i) la mise en forme du deuxième matériau à une dimension prédéterminée et ii) la fixation du deuxième matériau à l'au moins une partie de la surface externe du cadre annulaire ; et/ou
b) au moins une partie du deuxième matériau formé étant disposée sur un deuxième matériau perforé ayant une première surface et une seconde surface opposée, avant l'étape de fixation, et le deuxième matériau étant disposé sur la première surface et/ou la seconde surface du deuxième matériau perforé ; éventuellement, le deuxième matériau étant disposé entre deux couches du deuxième matériau perforé et les deux couches du deuxième matériau perforé étant au moins partiellement couplées l'une à l'autre ; et/ou
c) le procédé comprenant la disposition d'une deuxième couche auxiliaire sur au moins une partie de la première surface du deuxième matériau et/ou sur au moins une partie de la seconde surface du deuxième matériau.

16. Procédé selon l'une quelconque des revendications 12 à 15,
a) l'électrofilage étant effectué par cyclage ; et/ou
b) pendant l'électrofilage de l'au moins une partie de la pluralité de fibres (1304) pour former le premier matériau et/ou le deuxième matériau et/ou le troisième matériau, l'au moins une partie de la surface interne du cadre annulaire (1200) et/ou l'au moins une partie de la surface externe du cadre annulaire et/ou l'au moins une partie des premier, deuxième et/ou troisième mandrins prédéterminés étant positionnés à une certaine distance de l'au moins une filière d'extrusion de telle sorte que la distance varie pendant l'électrofilage afin de former une ou plusieurs pluralités de couches dans au moins une partie du premier matériau et/ou du deuxième matériau et/ou du troisième matériau.

17. Procédé selon l'une quelconque des revendications 12 à 16,
a) l'au moins une filière comprenant une aiguille ; ou
b) l'au moins une filière faisant partie d'un ensemble comprenant une pluralité de filières, l'ensemble comprenant éventuellement une pluralité de filières sans aiguille.
